# EUROPÄISCHE PATENTSCHRIFT

(11) **EP 1 406 878 B1**
(45) Veröffentlichungstag und Bekanntmachung des Hinweises auf die Patenterteilung: **07.06.2006**
(21) Anmeldenummer: 02751042.9
(22) Anmeldetag: 19.06.2002
(51) Int. Cl.: C07D 253/06, A61K 31/53, A61P 9/10, A61P 37/08, C07D 401/12

(54) **4-(BENZYLIDEN-AMINO)-3-(METHYLSULFANYL) -4H-(1, 2, 4) TRIAZIN-5-ON DERIVATE MIT PDE -IV HEMMENDER UND TNF-ANTAGONISTISCHER WIRKUNG ZUR BEHANDLUNG VON HERZKRANKHEITEN UND ALLERGIEN**
4-(BENZYLIDENE-AMINO)-3-(METHYLSULFANYL)-4H-(1, 2, 4) TRIAZIN-5-ONE DERIVATIVES HAVING A PDE-IV INHIBITING AND TNF-ANTAGONISTIC EFFECT FOR THE TREATMENT OF CARDIAC DISEASES AND ALLERGIES
DERIVES DE 4-(BENZYLIDENE-AMINO)-3-(METHYLSULFANYLE)-4H-(1, 2, 4) TRIAZIN-5-ONE A EFFET INHIBITEUR DE PDE-IV ET ANTAGONISTE DE TNF POUR LE TRAITEMENT DES MALADIES CARDIAQUES ET DES ALLERGIES

(30) Priorität: 18.07.2001 DE 10135009; 15.11.2001 DE 10156229
(43) Veröffentlichungstag der Anmeldung: 14.04.2004
(73) Patentinhaber: Merck Patent GmbH, 64293 Darmstadt (DE)
(72) Erfinder: EGGENWEILER, Hans-Michael, 64287 Darmstadt (DE); WOLF, Michael, 64297 Darmstadt (DE); BEIER, Norbert, 64354 Reinheim (DE); LEIBROCK, Joachim, 64319 Pfungstadt (DE); SCHELLING, Pierre, 64367 Mühltal (DE); GASSEN, Michael, 80796 München (DE); EHRING, Thomas, 42853 Remscheid (DE)
(86) Internationale Anmeldenummer: PCT/EP2002/006742
(87) Internationale Veröffentlichungsnummer: WO 2003/008392

(56) Entgegenhaltungen:
- EP-A- 0 763 534
- WO-A-02/09715
- WO-A-98/11075
- FR-A- 1 519 180
- FR-A- 1 547 854
- JP-A- 61 134 389
- US-A- 4 036 632
- US-A- 4 346 220
- DATABASE CA [Online] CHEMICAL ABSTRACTS SERVICE, COLUMBUS, OHIO, US; STAICU, SORIN ALEXANDER ET AL: "1,2,4-Triazin-5-one derivatives with herbicidal properties" retrieved from STN Database accession no. 95:115615 XP002210726 & RO 66 584 B (COMBINATUL CHIMIC "VICTORIA", ROM.) 15. Juni 1979 (1979-06-15)
- DATABASE CA [Online] CHEMICAL ABSTRACTS SERVICE, COLUMBUS, OHIO, US; SAUTER, FRITZ ET AL: "Alkylation of 4-amino-3-mercapto-1,2,4-triazin-5(4H)-one s and oxidation o the products" retrieved from STN Database accession no. 107:115563 XP002210727 & J. CHEM. RES., SYNOP., Bd. 9, 1986, Seiten 320-321,
- EL-GENDY, Z. ET AL.: "SYNTHESIS OF HETEROBICYCLIC NITROGEN SYSTEMS BEARING THE 1,2,4-TRIAZINE MOIETY AS ANTI-HIV AND ANTICANCER DRUGS, PART III" PHARMAZIE, Bd. 56, Nr. 5, 2001, Seiten 376-383, XP001094026

## Beschreibung

Die Erfindung betrifft Verbindungen der Formel I 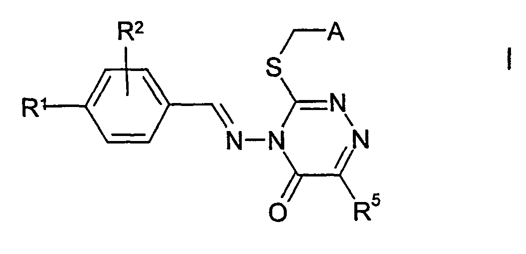 worin
- R¹, R²: jeweils unabhängig voneinander H, OH, OR⁶, SR⁶, SOR⁶, SO₂R⁶, Hal oder zusammen auch -O-CH₂-O-,
- A: durch R³ und R⁴ substituiertes Phenyl, 2-, 3- oder 4-Pyridyl, 4- oder 5-Pyrimidyl, 3- oder 4-Pyridazyl, 2- oder 3- Pyrazinyl,
- R³, R⁴: jeweils unabhängig voneinander H, OH, OR⁶, SR⁶, SOR⁶, SO₂R⁶, R⁶, Hal oder zusammen auch -O-CH₂-O-,
- R⁵: H oder Alkyl mit 1 bis 10 C-Atomen,
- R⁶: Alkyl mit 1 bis 10 C-Atomen, das durch 1 bis 5 F- und/oder Cl- Atome substituiert sein kann,
Cycloalkyl mit 3-7 C-Atomen, Alkylencycloalkyl mit 5-10 C- Atomen oder Alkenyl mit 2-8 C-Atomen,
- Hal: F, Cl, Br oder I
bedeuten,
sowie deren physiologisch unbedenklichen Salze und Solvate.

Ähnliche Verbindungen sind bereits bekannt (z.B. CAS Reg. No. 292057-55-7). Die erfindungsgemäßen Verbindungen unterscheiden sich jedoch von den bekannten in der Art und der Lage der Substituenten.

Die folgenden Dokumente offenbaren strukturell verwandte Verbindungen:
- D1:: US-A-4 036 632 (BAYER AG (DE)) 19. Juli 1977 (1977-07-19)
- D2:: FR-A-1 519 180 (BAYER AG (DE)) 29. März 1968 (1968-03-29)
- D3:: JP 61 134389 A (SHIONOGI & CO LTD (JP)) 21. Juni 1986 (1986-06-21)
- D4:: US-A-4 346 220 (DU PONT (US)) 24: August 1982 (1982-08-24)
- D5:: FR-A-1 547 854 (DU PONT (US)) 29. November 1968 (1968-11-29)
- D6:: DATABASE CA [Online] CHEMICAL ABSTRACTS SERVICE, COLUMBUS, OHIO, US; STAICU, SORIN ALEXANDER ET AL: '1,2,4-Triazin-5-one derivatives with herbicidal properties' retrieved from STN Database accession no. 95:115615 XP002210726 & RO 66 584 B (COMBINATUL CHIMIC 'VICTORIA', ROM.) 15. Juni 1979 (1979-06-15)
- D7:: DATABASE CA [Online] CHEMICAL ABSTRACTS SERVICE, COLUMBUS, OHIO, US; SAUTER, FRITZ ET AL: 'Alkylation of 4-amino-3-mercapto-1,2,4-triazin-5(4H)-one s and oxidation o the products' retrieved from STN Database accession no. 107:115563, XP002210727 & J. CHEM. RES., SYNOP. (1986), (9), 320-321
- D8:: EL-GENDY, Z. ET AL: 'Synthesis of heterobicyclic nitrogen systems bearing the 1,2,4-triazine moiety as anti-HIV and anticancer drugs, part III' PHARMAZIE (2001), 56(5), 376-383, XP001094026
- D9:: EP-A-0 763 534 (MERCK PATENT GMBH (DE)) 19. März 1997 (1997-03-19), in der Anmeldung erwähnt
- D10:: WO 98 11075 A (DU PONT MERCK PHARMA (US)) 19. März 1998 (1998-03-19)

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und Solvate bei guter Verträglichkeit sehr wertvolle pharmakologische Eigenschaften besitzen.

Insbesondere zeigen sie eine selektive Phosphodiesterase IV-Hemmung, die mit einer intrazellulären Erhöhung von cAMP verbunden ist (N. Sommer et al., Nature Medicine, **1**, 244-248 (1995)).
Die PDE IV-Hemmung kann z.B. analog C.W. Davis in Biochim. biophys. Acta **797**, 354-362 (1984) nachgewiesen werden.

Die erfindungsgemäßen Verbindungen können zur Behandlung von asthmatischen Erkrankungen eingesetzt werden. Die antiasthmatische Wirkung der PDE IV-Hemmer ist z.B. von T.J. Torphy et al. in Thorax, **46**, 512-523 (1991) beschrieben und kann z. B. nach der Methode von T. Olsson, Acta allergologica **26,** 438-447 (1971), bestimmt werden.

Da cAMP knochenabbauende Zellen hemmt und knochenaufbauende Zellen stimuliert (S. Kasugai et al., M 681 und K. Miyamoto, M 682, in Abstracts of the American Society for Bone and Mineral Research 18^{th} Annual Meeting, 1996), können die erfindungsgemäßen Verbindungen zur Behandlung von Osteoporose eingesetzt werden.

Weiterer Gegenstand der Erfindung ist daher die Verwendung der Verbindungen der Formel I und/oder ihre physiologisch unbedenklichen Salze und Solvate zur Herstellung eines Arzneimittels zur Behandlung und Prophylaxe von Krankheiten, die durch einen zu geringen cAMP-Spiegel verusacht werden und/oder durch eine Erhöhung des cAMP-Spiegels beeinflußt werden können.

Die Verbindungen zeigen außerdem eine antagonistische Wirkung auf die Produktion von TNFα (Tumor Nekrose Faktor) und eignen sich daher zur Behandlung von allergischen und entzündlichen Krankheiten, Autoimmunkrankheiten, wie z.B. rheumatoide Arthritis, multiple Sklerose, Morbus Crohn, Diabetes mellitus oder ulzerative Kolitis, Transplantatabstoßungsreaktionen, Kachexie und Sepsis.
Die antiinflammatorische Wirkung der erfindungsgemäßen Substanzen und ihre Wirksamkeit zur Behandlung von z.B. Autoimmunerkrankungen wie multipler Sklerose oder rheumatoider Arthritis, kann analog den Methoden von N. Sommer et al., Nature Medicine **1,** 244-248 (1995) oder L. Sekut et al., Clin. Exp. Immunol. **100,** 126-132 (1995) bestimmt werden.

Die Verbindungen können zur Behandlung von Kachexie eingesetzt werden. Die anti-kachektische Wirkung kann in TNF-abhängigen Modellen der Kachexie geprüft werden (P. Costelli et al., J. Clin. Invest. **95,** 2367ff. (1995); J.M. Argiles et al., Med. Res. Rev. **17**, 477ff. (1997)).

PDE IV-Inhibitoren können auch das Wachstum von Tumorzellen hemmen und sind deshalb für die Tumortherapie geeignet (D. Marko et al., Cell Biochem. Biophys. **28,** 75ff. (1998)). Die Wirkung von PDE IV-Hemmern bei der Tumorbehandlung ist z.B. in der WO 95 35 281, WO 95 17 399 oder WO 96 00 215 beschrieben.

Weiterer Gegenstand der Erfindung ist daher die Verwendung der Verbindungen der Formel I und/oder ihre physiologisch unbedenklichen Salze und Solvate zur Herstellung eines Arzneimittels zur Behandlung und Prophylaxe von Krankheiten, die durch eine Überproduktion von Tumor Nekrose Faktor (TNF) verursacht werden und/oder durch eine Verringerung der Produktion von TNF beeinflußt werden können.

PDE IV-Inhibitoren können die Mortalität in Modellen für Sepsis verhindern und eignen sich daher für die Therapie von Sepsis (W. Fischer et al., Biochem. Pharmacol. **45,** 2399ff. (1993)).

Sie können weiterhin zur Behandlung von Gedächtnisstörungen, Atherosklerose, atopische Dermatitis und AIDS eingesetzt werden.

Die Wirkung von PDE IV-Hemmern bei der Behandlung von Asthma, entzündlichen Erkrankungen, Diabetes mellitus, atopischer Dermatitis, Psoriasis, AIDS, Kachexie, Tumorwachstum oder Tumormetastasen ist z.B. in der EP 77 92 91 beschrieben.

Die Verbindungen der Formel 1 weisen als Inhibitoren der PDE IV lsozyme breite therapeutische Anwendungsmöglichkeiten auf, da die PDE IV Familie von lsozymen eine wesentliche Rolle in der Physiologie aller Säuger spielt. Die PDE IV lsozyme bewirken die intracellulläre Hydrolyse von Adenosin 3', 5'-Monophosphate (cAMP) in pro-inflammatorischen Leukozyten. cAMP wiederum ist verantwortlich für Mediation der Wirkung zahlreicher Hormone im Körper.
Es existiert eine Fülle von Literatur, die die Wirkungen von PDE Inhibitoren auf verschiedenste Inflammatorische Zell-Antworten beschreibt, die zusätzlich zur Erhöhung des cAMP-Spiegels auch die Hemmung der Superoxid-Produktion, Degranulation, Chemotaxis und Freisetzung von Tumor Necrose Faktor (TNF) in Eosinophilen, Neutrophilen und Monozyten beinhalten.

Ein weiterer Gegenstand der Erfindung ist daher die Verwendung der Verbindungen der Formel I und/oder deren physiologisch verträglichen Salze und/oder Solvate, zur Herstellung eines Medikamentes zur Behandlung oder Prophylaxe von Krankheiten, die durch Störungen der Regulation der Aktivierung und Degranulation von humanen Eosinophilen hervorgerufen werden.

Die Verbindungen der Formel I können als Arzneimittelwirkstoffe in der Human- und Veterinärmedizin eingesetzt werden. Ferner können sie als Zwischenprodukte zur Herstellung weiterer Arzneimittetwirkstoffe eingesetzt werden.

Bevorzugt können die Verbindungen der Formel I auch zusammen mit einem oder mehreren bekannten PDE-IV Inhibitoren verwendet werden.

Vorzugsweise werden die Verbindungen der Formel I zusammen mit einem oder mehreren der in den folgenden Dokumenten veröffentlichten PDE-IV Inhibitoren verwendet: EP 0763534, WO 99/65880, WO 99/08047, WO 98/06704, WO 00/59890, DE 19604388, DE 19932315, EP 0723962, EP 0738715.

Die Erfindung betrifft außerdem die Verwendung der Verbindungen der Formel I als PDE-IV-Inhibitoren zur Behandlung von Herzmuskelerkrankungen.

In westlichen Ländern ist die koronare Herzkrankheit die häufigste Todesursache. Bei einer kritisch verengten Koronararterie kann der verminderte Blutstrom zu Myokardischämie führen. In Abhängigkeit von dem Schweregrad der vorhergehenden ischämischen Periode hat das Einsetzen der Reperfusion eine reversible oder irreversible Myokardverletzung zur Folge, die durch eine langanhaltende Depression oder einen irreversiblen Verlust der Kontraktionsfunktion gekennzeichnet ist. Je nach der Größe des betroffenen Myokardbereiches kann es zu akutem oder chronischem Herzversagen kommen.
Ein besonderes klinisches Problem in dem oben beschriebenen Szenario ist das Einsetzen von Restenose nach einer zunächst erfolgreichen Reperfusionsintervention durch PTCA (perkutane transluminale Koronarangioplastie), selbst nach Stentimplantation, Thrombolyse oder Einsetzen eines aorto-koronaren Bypass.
Tierexperimentelle und klinische Studien deuten darauf hin, daß entzündliche Prozesse bei den verschiedenen oben erwähnten Herzkrankheiten, z.B. bei koronarer Herzkrankheit selbst, bei reversibler oder irreversibler Myokardischämie/Reperfusionsschäden, bei akutem oder chronischem Herzversagen und bei Restenose einschließlich Instent-Restenose und Stent-in-Stent-Restenose, eine verursachende Rolle spielen. An diesen entzündlichen Prozessen sind residente und eindringende Makrophagen sowie neutrophile Zellen und TH1- und TH2-Helferzellen beteiligt. Diese Leukocytenreaktion führt zu einem charakteristischen Cytokin-Muster unter Beteiligung von TNF-α, IL-1β, lL-2 und lL-6 sowie IL-10 und IL-13 (Pulkki KJ: Cytokines and cardiomyocyte death, Ann.Med. **1997** *29*: 339-343. Birks EJ, Yacoub MH: The role of nitric oxide and cytokines in heart failure. Coron.Artery.Dis. **1997** *8***:** 389-402).
Es wurde gezeigt, daß diese Spezies bei menschlichen Patienten mit Myokardischämie gebildet werden. Tiermodelle zeigen, daß die Cytokinproduktion mit dem Eindringen von periphären Makrophagen und neutrophilen Zellen, die die Schädigung in das noch intakte Myokard weiterverbreiten können, korreliert.
Die Hauptrolle bei der Cytokinreaktion spielt jedoch der TNF-α, der entzündliche und proapoptotische Reaktionen integriert und darüber hinaus einen direkten negativen ionotropischen Effekt auf Herzmuskelzellen hat (Ceconi C, Curello S, Bachetti T, Corti A, Ferrari R: Tumor necrosis factor in congestive heart failure: a mechanism of disease for the new millenium? Pro.Cardiovas.Dis. **1998** *41*: 25-30. Mann DL: The effect of tumor necrosis factor-alpha on cardiac structure and function: a tale of two cytokines. J.Card.Fail. **1996** *2*: S165-S175. Squadrito F, Altavilla D, Zingarelli B, et al.: Tumor necrosis factor involvement in myocardial ischaemia-reperfusion injury. Eur.J.Pharmacol. **1993** *237*: 223-230).

In Tiermodellen des Herzinfarkts wurde gezeigt, daß es während der Reperfusionsphase zu einer schnellen Freisetzung von TNF-α kommt (Herskowitz A, Choi S, Ansari AA, Wesselingh S: Cytokine mRNA expression in postischemic/reperfused myocardium. Am.J.Pathol. **1995** *146*: 419-428) und daß die schützende Wirkung von Arzneimitteln wie Dexamethason (Arras M, Strasser R, Mohri M, et al.: Tumor necrosis factor-alpha is expressed by monocytes/macrophages following cardiac microembolization and is antagonized by cyclosporine, Basic.Res.Cardiol. **1998** *93*:97-107), Cyclosporin A (Arras M, Strasser R, Mohri M, et al.: Tumor necrosis factor-alpha is expressed by monocytes/macrophages following cardiac microembolization and is antagonized by cyclosporine, Basic.Res.Cardiol. **1998** *93*:97-107, Squadrito F, Altavilla D, Squadrito G, et al.: Cyclosporin-A reduces leukocyte accumulation and protects against myocardial ischaemia reperfusion injury in rats. Eur.J.Pharmacol. **1999** *364*: 159-168) oder Clorichromen (Squadrito F, Altavilla D, Zingarelli B, et al.: The effect of cloricromene, a coumarine derivative, on leukocyte accumulation, myocardial necrosis and TNF-alpha production in myocardial ischaemia-reperfusion injury. Life Sci. **1993** *53*: 341-355) mit einer Absenkung des im Kreislauf befindlichen TNF-α einhergeht.

Die vorliegende Erfindung betrifft daher auch die Verwendung der Verbindungen der Formel I und/oder ihre physiologisch unbedenklichen Salze und Solvate zur Herstellung eines Arzneimittels zur Behandlung und Prophylaxe von Krankheiten, die durch eine Verringerung der Produktion von Tumor Nekrose Faktor (TNF) beeinflußt werden können.

Die PDE IV-Inhibitoren der Formel I sind potentielle Antagonisten der Produktion von Makrophagen und T-Zellen-Cytokinen. Sie hemmen darüber hinaus die Proliferation von T-Zellen. Als Folge davon kann eine PDE IV-Inhibierung bei Herzmuskelerkrankungen, bei denen ein kausaler Zusammenhang mit der Produktion von Cytokinen und entzündlichen Prozessen besteht, eine vorteilhafte Wirkung haben.

Die Verwendung der Verbindungen der Formel I und/oder ihre physiologisch unbedenklichen Salze und Solvate zur Herstellung eines Arzneimittels zur Behandlung und Prophylaxe von Krankheiten, die durch eine Überproduktion von Makrophagen und T-Zellen verusacht wird und/oder durch eine Verrringerung der Makrophagen- und T-Zell-Produktion beeinflußt werden können ist ebenfalls Gegenstand der vorliegenden Erfindung.

Weiterhin betrifft die vorliegende Erfindung die Verwendung der Verbindungen der Formel I und/oder ihre physiologisch unbedenklichen Salze und Solvate zur Herstellung eines Arzneimittels zur Behandlung und Prophylaxe von Krankheiten, die durch übermäßige Proliferation der T-Zellen verusacht werden und/oder durch eine Hemmung der Proliferation der T-Zellen beeinflußt werden können.

Im Vergleich zu PDE III-Inhibitoren und dem frühen PDE IV-Inhibitor Rolipram zeigen bevorzugte PDE IV-Inhibitoren der Formel I keine hämodynamischen Nebenwirkungen, die bei der Behandlung der meisten Herzkreislauferkrankungen dosisbegrenzend sein könnten.

Der Erfindung liegt die Aufgabe zugrunde, neue Verwendungen für Verbindungen mit wertvollen Eigenschaften, insbesonderen von solchen, die sich für die Herstellung von Medikamenten eignen, zu finden.

Es wurde gefunden, daß die PDE-IV-Inhibitoren der Formel I und ihre Salze und Solvate bei der Behandlung von Herzmuskelerkrankungen sehr wertvolle pharmakologische Eigenschaften zeigen und gleichzeitig gut verträglich sind.

Die bevorzugten Verbindungen bewirken eine selektive Hemmung von Phosphodiesterase IV, die mit einem intrazellulären Anstieg der cAMP-Konzentration assoziiert wird (N. Sommer et al., Nature Medicine, **1**, 244-248 (1995)).
Die Inhibierung von PDE IV läßt sich beispielsweise gemäß C.W. Davis, Biochim. Biophys. Acta **797**, 354-362 (1984), zeigen.
Man mißt die Affinität der erfindungsgemäßen Verbindungen für Phosphodiesterase IV, indem man ihre IC₅₀-Werte (die Inhibitorkonzentration, die erforderlich ist, um die Enzymaktivität um 50% zu hemmen) bestimmt.

Weiterer Gegenstand der vorliegenden Anmeldung ist daher die Verwendung der Verbindugen der Formel und/oder ihre physiologisch unbedenklichen Salze und Solvate zur Herstellung eines Arzneimittels zur Behandlung und Prophylaxe von Krankheiten, die durch eine Erhöhung des cAMP-Spiegels beeinflußt werden können.

Die Erfindung stellt vorzugsweise die Verwendung der oben erwähnten Verbindungen zur Herstellung eines Medikaments zur Behandlung von Herzmuskelerkrankungen bereit, die entzündliche und immunologische Charakteristika aufweisen.

Ganz besonders bevorzugt stellt die Erfindung die Verwendung der oben erwähnten Verbindungen zur Herstellung eines Medikaments zur Behandlung von koronarer Herzkrankheit, reversibler oder irreversibler Myokardischämie/Reperfusionsschäden, akutem oder chronischem Herzversagen, dekompensierter Herzinsuffizienz (congestive heart failure, CHF) und Restenose einschließlich Instent-Restenose und Stent-in-Stent-Restenose bereit.

Ferner eignen sich die Verbindungen der Formel und/oder ihre Salze und/oder Solvate zur Herstellung eines Arzneimittels zur Prophylaxe und Behandlung von ventrikulärer Umbildung (venticular remodeling) nach Infarkt oder dekompensierter Herzinsuffizienz (congestive heart failure, CHF) unterschiedlicher Ausprägung.

Die Zubereitungen zur Behandlung der erwähnten Erkrankungen können in Human- und Veterinärmedizin als Medikamente eingesetzt werden. Mögliche Trägerstoffe sind zur enteralen (z.B. oralen) oder parenteralen Verabreichung oder topischen Anwendung geeignete organische oder anorganische Substanzen, die nicht mit den neuen Verbindungen reagieren, beispielsweise Wasser, Pflanzenöle, Benzylalkohole, Alkylenglykole, Polyethylenglykole, Glycerintriacetat, Gelatine, Kohlenhydrate wie Lactose oder Stärke, Magnesiumstearat, Talkum und Vaseline. Insbesondere werden Tabletten, Pillen, Dragees, Kapseln, Pulver, Granulate, Sirups, Säfte oder Tropfen zur oralen Verabreichung, Zäpfchen zur rektalen Verabreichung, Lösungen, vorzugsweise ölige oder wäßrige Lösungen, und weiterhin Suspensionen, Emulsionen oder Implantate zur parenteralen Verabreichung und Salben, Cremes oder Pulver zur topischen Anwendung eingesetzt. Es ist auch möglich, die neuen Verbindungen zu lyophilisieren und die so erhaltenen Lyophilisate zum Beispiel für die Herstellung von Injektionszubereitungen zu verwenden. Die aufgezeigten Zubereitungen können sterilisiert werden und/oder Hilfsstoffe wie z.B. Gleitmittel, Konservierungsstoffe, Stabilisierungsmittel und/oder Netzmittel, Emulgatoren, Salze zum Einstellen des osmotischen Drucks, Puffer, Farbstoffe, Geschmackstoffe und/oder einen oder mehrere weitere Wirkstoffe, z.B. ein oder mehrere Vitamine, enthalten.

Bei diesen Indikationen werden die Substanzen im allgemeinen vorzugsweise in Dosen von etwa 1 bis 500 mg, insbesondere von 5 bis 100 mg pro Dosiseinheit verabreicht. Die Tagesdosis beträgt vorzugsweise von etwa 0,02 bis 10 mg/kg Körpergewicht. Die spezifische Dosis für den jeweiligen Patienten hängt jedoch von einer Reihe von Faktoren ab, beispielsweise von der Wirksamkeit der verwendeten Verbindung, vom Alter, Körpergewicht, allgemeinen Gesundheitszustand, Geschlecht, von der Ernährung, von Verabreichungszeitpunkt und -weg, von der Ausscheidungsrate, der Medikamentenkombination und der Schwere der Krankheit, gegen die die Therapie eingesetzt wird. Eine orale Verabreichung ist bevorzugt.

Gegenstand der Erfindung sind dementsprechend die Verbindungen der Formel I sowie ein Verfahren zur Herstellung von Verbindungen der Formel I nach Anspruch 1 sowie deren Salze und Solvate, dadurch gekennzeichnet, daß man eine Verbindung der Formel II 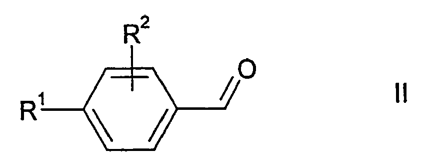 worin
R¹ und R² die angegebenen Bedeutungen haben,
mit einer Verbindung der Formel lll 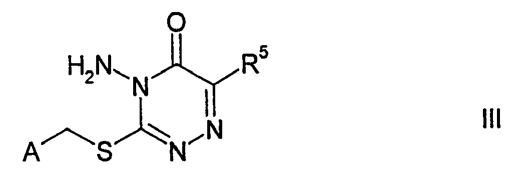 worin
A und R⁵ die oben angegebenen Bedeutungen haben,
umsetzt,
und/oder daß man eine basische Verbindung der Formel I durch Behandeln mit einer Säure in eines ihrer Salze überführt.

Die neuen Verbindungen der Formel III sind ebenfalls Gegenstand der Erfindung.

Unter Solvaten der Verbindungen der Formel I werden Anlagerungen von vorzugsweise inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Alkoholate.

Vor- und nachstehend haben die Reste R¹, R², A, R³, R⁴, R⁵ und R⁶ die bei den Formeln I, II und III angegebenen Bedeutungen, sofern nicht ausdrücklich etwas anderes angegeben ist.

R⁶ bedeutet vorzugsweise Alkyl, weiter bevorzugt durch 1 bis 5 Fluor- und/oder Chloratome substituiertes Alkyl, weiter bevorzugt Cycloalkyl. In den vorstehenden Formeln ist Alkyl vorzugsweise unverzweigt und hat 1, 2, 3, 4, 5, 6, 7, 8, 9 oder 10 C-Atome, vorzugsweise 1, 2, 3, 4, 5 oder 6 C-Atome und bedeutet vorzugsweise Methyl, Ethyl, Trifluormethyl, Pentafluorethyl oder Propyl, weiterhin bevorzugt Isopropyl, Butyl, Isobutyl, sek.-Butyl oder tert.-Butyl, aber auch n-Pentyl, neo-Pentyl, Isopentyl oder n-Hexyl. Besonders bevorzugt ist Methyl, Ethyl, Trifluormethyl, Propyl, Isopropyl, Butyl, n-Pentyl, n-Hexyl oder n-Decyl.

Cycloalkyl hat vorzugsweise 3-7 C-Atome und steht bevorzugt für Cyclopropyl und Cyclobutyl, weiterhin bevorzugt für Cyclopentyl oder Cyclohexyl, ferner auch für Cycloheptyl, besonders bevorzugt ist Cyclopentyl.

Alkenyl steht vorzugsweise für Allyl, 2- oder 3-Butenyl, Isobutenyl, sek.-Butenyl, ferner bevorzugt ist 4-Pentenyl, iso-Pentenyl oder 5-Hexenyl.

Alkylen ist vorzugsweise unverzweigt und bedeutet bevorzugt Methylen oder Ethylen, ferner bevorzugt Propylen oder Butylen.

Alkylencycloalkyl hat vorzugsweise 5-10 C-Atome und steht bevorzugt für Methylencyclopropyl, Methylencyclobutyl, weiterhin bevorzugt für Methylencyclopentyl, Methylencyclohexyl oder Methylencycloheptyl, ferner auch für Ethylencyclopropyl, Ethylencyclobutyl, Ethylencyclopentyl, Ethylencyclohexyl oder Ethylencycloheptyl, Propylencyclopentyl, Propylencyclohexyl, Butylencyclopentyl oder Butylencyclohexyl.

Hal bedeutet vorzugsweise F, Cl oder Br, aber auch 1.

Die Reste R¹ und R² können gleich oder verschieden sein. R² steht vorzugsweise in der 3- Position des Phenylrings. Die Reste R¹ und R² bedeuten unabhängig voneinander beispielsweise Hydroxy, -S-CH₃, -SO-CH₃, -SO₂CH₃, F, Cl, Br oder I oder zusammeli Methylendioxy. Bevorzugt stehen sie aber jeweils für Methoxy, Ethoxy, Propoxy, Cyclopentoxy, oder aber für Fluor-, Difluor-, Trifluormethoxy, 1-Fluor-, 2-Fluor-, 1,2-Difluor-, 2,2-Difluor-, 1,2,2-Trifluor- oder 2,2,2-Trifluorethoxy.

R¹ steht besonders bevorzugt für Methoxy, Ethoxy, Cyclopentoxy oder Isopropoxy.
R² steht besonders bevorzugt für Methoxy oder Ethoxy.

A bedeutet vorzugsweise Phenyl, 2-, 3- oder 4-Pyridyl, 4- oder 5-Pyrimidyl. Besonders bevorzugt Phenyl, 2-, 3- oder 4-Pyridyl, insbesondere Phenyl oder 3-Pyridyl.

R³ und R⁴ stehen am Ring A bevorzugt in ortho- und para-Position zur Gruppe -CH₂S-. Die Reste R³ und R⁴ sind auch im Falle, daß A einen Heteroaromaten darstellt, stets an ein Kohlenstoff-Atom im Ring gebunden. Besonders bevorzugt bedeuten R³ und R⁴ in diesem Fall jeweils ein H-Atom.

R³ und R⁴ nehmen unabhängig voneinander vorzugsweise die Bedeutung von R⁶ oder eine der für R¹ und R² genannten Bedeutungen an. Besonders bevorzugt bedeutet R³ Methoxy, Ethoxy, Propoxy, Cyclopentoxy, oder aber für Fluor-, Difluor-, Trifluormethoxy, 1-Fluor-, 2-Fluor-, 1,2-Difluor-, 2,2-Difluor-, 1,2,2-Trifluor- oder 2,2,2-Trifluorethoxy. R⁴ bedeutet besonders bevorzugt Alkoxy oder Alkyl, insbesondere Methoxy, Ethoxy, Propoxy, Cyclopentoxy oder Methyl, Ethyl, Trifluormethyl, Propyl, Isopropyl, Butyl, n-Pentyl, n-Hexyl oder n-Decyl.

R⁵ bedeutet vorzugsweise jeweils unabhängig voneinander H oder Methyl, insbesondere Methyl.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis If ausgedrückt werden, die der Formel I entsprechen und worin die nicht näher bezeichneten Reste die bei der Formel I angegebene Bedeutung haben, worin jedoch
- in Ia: R¹ und R² jeweils unabhängig voneinander OR⁶ bedeuten;
- in Ib: R¹ und R² jeweils unabhängig voneinander OR⁶,
R⁶ Alkyl mit 1-10 C-Atomen oder Cycloalkyl mit 3-7 C-Atomen bedeuten;
- in Ic: R¹ und R² jeweils unabhängig voneinander OR⁶,
R⁶ Alkyl mit 1-10 C-Atomen oder Cycloalkyl mit 3-7 C-Atomen,
A Phenyl, 2-, 3- oder 4-Pyridyl, 4- oder 5-Pyrimidyl,
R³, R⁴ jeweils unabhängig voneinander R⁶, H, Cl, CF₃ oder OR⁶ bedeuten;
- in Id: R¹ und R² jeweils unabhängig voneinander OR⁶,
R⁶ Alkyl mit 1-10 C-Atomen oder Cycloalkyl mit 3-7 C-Atomen,
A Phenyl, 2-, 3- oder 4-Pyridyl, 4- oder 5-Pyrimidyl,
R³, R⁴ jeweils unabhängig voneinander H, Cl, F, CF₃ oder OR⁶,
R⁵ H oder Methyl bedeuten;
- in Ie: R¹ und R² jeweils unabhängig voneinander OR⁶,
R⁶ Alkyl mit 1-10 C-Atomen oder Cycloalkyl mit 3-7 C-Atomen,
A Phenyl, 2-, 3- oder 4-Pyridyl, 4- oder 5-Pyrimidyl,
R³, R⁴ jeweils unabhängig voneinander H, Cl, F, CF₃ oder OR⁶,
R⁵ Methyl bedeuten;

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Verbindungen der Formel I können vorzugsweise erhalten werden, indem man Verbindungen der Formel II mit Verbindungen der Formel III umsetzt.

Die Ausgangsstoffe der Formeln II und III sind teilweise bekannt (z.B. U.S. 3,905,801). Sofern sie nicht bekannt sind, können sie nach an sich bekannten Methoden hergestellt werden.

Im einzelnen erfolgt die Umsetzung der Verbindungen der Formel II mit den Verbindungen der Formel III in Gegenwart oder Abwesenheit eines vorzugsweise inerten Lösungsmittels bei Temperaturen zwischen etwa -20 und etwa 150°, vorzugsweise zwischen 20 und 100°.

Als Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Bei der Umsetzung von Verbindungen der Formel II mit Verbindungen der Formel III können wasserentziehende Mittel eingesetzt werden, wie sie für ähnliche Umsetzungen von Carbonyl- mit Aminoverbindungen bekannt sind, um das Reaktionsgleichgewicht auf die Seite der Produkte zu verschieben. Beispielsweise können Silicagel, Molekularsieb, hygroskopische Salze, Lösungsmittel oder Säuren verwendet werden. Ebenso kann während der Reaktion gebildetes Wasser durch übliche Methoden wie Verdampfen oder Auskreisen aus dem Reaktionsgemisch entfernt werden. Zur Gleichgewichtsverschiebung ist es ferner ebenfalls möglich, ein Lösungsmittel zu verwenden, in dem zwar die Ausgangsverbindungen II und III gelöst werden, nicht aber die Verbindungen der Formel I, so daß gebildetes Produkt dem Gleichgewicht entzogen wird.

Der für die Umsetzung erforderliche pH-Wert kann in Anlehnung an für ähnliche Umsetzungen von Carbonyl- mit Aminoverbindungen gewählte pH-Werte eingestellt werden. Vorzugsweise eignet sich als Säurezusatz eine Carbonsäure, insbesondere Essigsäure.

Eine Base der Formel I kann mit einer Säure in das zugehörige Säureadditionssalz übergeführt werden, beispielsweise durch Umsetzung äquivalenter Mengen der Base und der Säure in einem vorzugsweise inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Sauten, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können, falls gewünscht, die freien Basen der Formel aus ihren Salzen mit Basen (z.B. Natrium- oder Kaliumhydroxid oder -carbonat) in Freiheit gesetzt werden.

Gegenstand der Erfindung sind Verbindungen der Formel I und ihre physiologisch unbedenklichen Salze und Solvate als Arzneimittel.

Gegenstand der Erfindung sind auch die Verbindungen der Formel I und ihre physiologisch unbedenklichen Salze und Solvate als Phosphodiesterase IV-Hemmer.

Gegenstand der Erfindung ist ferner die Verwendung der Verbindungen der Formel I und/oder ihrer physiologisch unbedenklichen Salze und/oder Solvate zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze und/oder Solvate.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, . Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder ein oder mehrere weitere Wirkstoffe enthalten, z.B. ein oder mehrere Vitamine.

Die Verbindungen der Formel I und ihre physiologisch unbedenklichen Salze und Solvate können bei der Bekämpfung von Krankheiten eingesetzt werden, wobei eine Erhöhung des cAMP(cyclo-Adenosin-monophosphat)-Spiegels erreicht wird, die zu Entzündungshemmung oder -verhinderung und Muskelentspannung führt. Besondere Verwendung können die erfindungsgemäßen PDE IV-Inhibitoren bei der Behandlung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS finden.

Dabei werden die erfindungsgemäßen Substanzen in der Regel vorzugsweise in Dosierungen entsprechend der Verbindung Rolipram zwischen 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

### Beispiel I: Wirkung der PDE IV-Inhibitoren der Formel I auf die Proliferation von T-Zellen

Aus dem Blut gesunder Spender werden periphere mononukleäre Blutzellen (peripheral blood mononuclear cells, PBMC) unter Anwendung der Lymphoprep-Gradientenmethode isoliert. 200000 PMBC/Vertiefung werden 5 Tage lang bei 37°C und 10% CO₂ in Mikrotiterplatten mit flachem Boden und 96 Vertiefungen in RPMI 1640-Kulturmedium mit 5% hitzeinaktiviertem Humanserum (AB-Pool) kultiviert. Die T-Zellen in der PBMC-Zubereitung werden selektiv mit einem monoklonalen Antikörper gegen CD3 stimuliert. Es werden jeweils drei Ansätze der Kulturen angefertigt, einschließlich einer Kontrollgruppe, die nicht behandelt wird. Die PDE IV-Inhibitoren der Formel I werden in DMSO auf eine Konzentration von 10⁻² M gelöst und mit Kulturmedium verdünnt. Die Kontrollkulturen werden der Inhibitorkonzentration entsprechend mit DMSO versetzt. 18 Stunden vor dem Ende des Assays wird den Kulturen ³H-Thymidin zugesetzt. Die Aufnahme von Radioaktivität in die Zellen wird dann in einem Beta-Counter gemessen. Die Daten von wenigstens drei unabhängigen Experimenten werden als prozentuale Inhibierung der Kontrolle (Mittelwert ± Standardabweichung) ohne Inhibitor berechnet. Aus diesen Daten wirde der IC₅₀-Wert bestimmt.

### Ergebnisse:

Die PDE IV-Inhibitoren der Formel I bewirken eine deutliche Verminderung der T-Zellen-Proliferatiori.

### Beispiel II: Wirkung der PDE IV-Inhibitoren der Formel I auf die CytokinProduktion in humanen peripheren monozytären Blutzellen

Aus dem Blut gesunder Spender werden periphere mononukleäre Blutzellen (peripheral blood mononuclear cells, PBMC) unter Anwendung der Lymphoprep-Gradientenmethode isoliert. 200000 PMBC/Vertiefung werden bei 37°C und 10% CO₂ in Mikrotiterplatten mit flachem Boden und 96 Vertiefungen in RPMI1640-Kulturmedium mit 5% hitzeinaktiviertem Humanserum (AB-Pool) kultiviert. Es werden jeweils drei Ansätze der Kulturen angefertigt, einschließlich einer Kontrollgruppe. Es werden 10⁻² M Lösungen der PDE IV-Inhibitoren der Formel I in DMSO angefertigt, die dann mit Kulturmedium verdünnt werden. Die Kontrollkulturen werden mit DMSO-Konzentrationen entsprechend der Inhibitorkonzentration versetzt. Das betreffende Cytokin wird stimuliert.
Die Kulturüberstände von drei unabhängigen Experimenten werden gepoolt, und die Cytokin-Aktivität im Überstand wird mit einem handelsüblichen ELISA-Testkit gemessen. Die Daten werden als prozentuale Inhibierung/Stimulation der Kontrollgruppe ohne Verbindung berechnet, und bei einer Stimulierung wird der entsprechende IC₅₀-Wert bzw. EC₅₀-Wert bestimmt.

### Ergebnis

Die PDE IV-Inhibitoren der Formel I bewirken eine deutlich verminderte Freisetzung von IL-2, IFN-γ, TNF-α und IL-12.

### Beispiel III: Wirkung der PDE IV-Inhibitoren der Formel I auf experimentelle Myokardinfarkte in Ratten

In Ratten bewirkt die Verbindung 5 bei intraperitonealer Verabreichung von 1, 3 bzw. 10 mg/kg 1 Stunde vor einem reversiblen Verschluß der linken Koronararterie eine signifikante, dosisabhängige Reduzierung der Infarktgröße von bis zu 38%. In Übereinstimmung mit diesem Schutz wird eine mittels ELISA gemessene Absenkung der TNF-alpha-Konzentration im Plasma beobachtet.

### Beispiel IV: Wirkung der PDE IV-Inhibitoren der Formel auf experimentelle Myokardinfarkte in Kaninchen

In narkotisierten Kaninchen, bei denen die Koronararterie (Seitenarm des Ramus circumflexus der linken Koronararterie) 30 Minuten lang verschlossen und anschließend 120 Minuten lang reperfundiert wird, hat die PDE IV-Inhibierung eine kardioprotektive Wirkung. Verglichen mit Placebo verminderten vor dem Koronarverschluß verabreichte PDE IV-Inhibitoren der Formel I die Infarktgröße. Die gefährdeten Regionen waren bei Verum- und Plazebogruppe vergleichbar. Man kann die kardioprotektive Wirkung nicht günstigen hämodynamischen Wirkungen zuschreiben, da während der Durchführung des Experiments Herzfrequenz und mittlerer Aortendruck konstant bleiben.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Die Angaben ortho (o), meta (m) und para (p) beziehen sich auf die an den Ringen befindliche Gruppe -CH₂S- bzw. -CHN-.

### Beispiel 1

0,272 g 4-Amino-3-mercapto-6-methyl-4*H*-[1,2,4]triazin-5-on (herstellbar nach A. Dornow, H. Menzel, P.Marx, Chem. Ber. 97, 2173 (1964)) wurden in 0,86 ml einer 2 N Lösung von Natriumhydroxid in Wasser suspendiert. Anschließend tropfte man eine Lösung von 0,218 g Benzylchlorid in Ethanol hinzu und erhitzte die Mischung für 30 min. auf 80°C. Der entstandene Niederschlag wurde abgesaugt, wodurch 4-Amino-3-benzylsulfanyl-6-methyl-4*H*-[1,2,4]triazin-5-on erhalten wurde.

Analog werden unter Verwendung der entsprechenden Vorstufen die folgenden Verbindungen der Formel Illa erhalten:

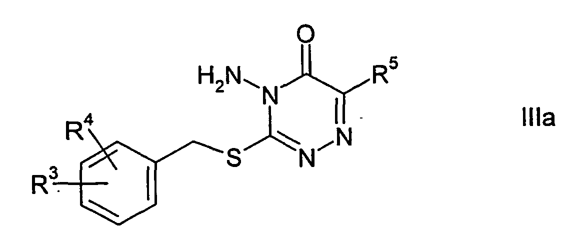

| | R³ | R⁴ | R⁵ |
|---|---|---|---|
| (2) | o-F | H | CH₃ |
| (3) | m-F | H | CH₃ |
| (4) | p-F | H | CH₃ |
| (5) | o-Cl | H | CH₃ |
| (6) | m-Cl | H | CH₃ |
| (7) | p-Cl | H | CH₃ |
| (8) | o-Cl | o-Cl | CH₃ |
| (9) | m-Cl | o-Cl | CH₃ |
| (10) | p-Cl | o-Cl | CH₃ |
| (11) | m-Cl | m-Cl | CH₃ |
| (12) | p-Cl | m-Cl | CH₃ |
| (13) | o-Cl | o-F | CH₃ |
| (14) | m-Cl | o-F | CH₃ |
| (15) | p-Cl | o-F | CH₃ |
| (16) | o-Cl | m-F | CH₃ |
| (17) | m-Cl | m-F | CH₃ |
| (18) | p-Cl | m-F | CH₃ |
| (19) | o-Cl | p-F | CH₃ |
| (20) | m-Cl | p-F | CH₃ |
| (21) | o-F | o-F | CH₃ |
| (22) | m-F | o-F | CH₃ |
| (23) | p-F | o-F | CH₃ |
| (24) | m-F | m-F | CH₃ |
| (25) | p-F | m-F | CH₃ |
| (26) | o-OCH₃ | H | CH₃ |
| (27) | m-OCH₃ | H | CH₃ |
| (28) | p-OCH₃ | H | CH₃ |
| (29) | o-OCH₃ | o-Cl | CH₃ |
| (30) | m-OCH₃ | o-Cl | CH₃ |
| (31) | p-OCH₃ | o-Cl | CH₃ |
| (32) | m-OCH₃ | m-Cl | CH₃ |
| (33) | p-OCH₃ | m-Cl | CH₃ |
| (34) | o-OCH₃ | o-F | CH₃ |
| (35) | m-OCH₃ | o-F | CH₃ |
| (36) | p-OCH₃ | o-F | CH₃ |
| (37) | o-OCH₃ | m-F | CH₃ |
| (38) | m-OCH₃ | m-F | CH₃ |
| (39) | p-OCH₃ | m-F | CH₃ |
| (40) | o-OCH₃ | p-F | CH₃ |
| (41) | m-OCH₃ | p-F | CH₃ |
| (42) | o-OCH₃ | o-OCH₃ | CH₃ |
| (43) | m-OCH₃ | o-OCH₃ | CH₃ |
| (44) | p-OCH₃ | o-OCH₃ | CH₃ |
| (45) | m-OCH₃ | m-OCH₃ | CH₃ |
| (46) | p-OCH₃ | m-OCH₃ | CH₃ |
| (47) | o-OH | H | CH₃ |
| (48) | m-OH | H | CH₃ |
| (49) | p-OH | H | CH₃ |
| (50) | o-OH | o-Cl | CH₃ |
| (51) | m-OH | o-Cl | CH₃ |
| (52) | p-OH | o-Cl | CH₃ |
| (53) | m-OH | m-Cl | CH₃ |
| (54) | p-OH | m-Cl | CH₃ |
| (55) | o-OH | o-F | CH₃ |
| (56) | m-OH | o-F | CH₃ |
| (57) | p-OH | o-F | CH₃ |
| (58) | o-OH | m-F | CH₃ |
| (59) | m-OH | m-F | CH₃ |
| (60) | p-OH | m-F | CH₃ |
| (61) | o-OH | p-F | CH₃ |
| (62) | m-OH | p-F | CH₃ |
| (63) | o-OH | o-OH | CH₃ |
| (64) | m-OH | o-OH | CH₃ |
| (65) | p-OH | o-OH | CH₃ |
| (66) | m-OH | m-OH | CH₃ |
| (67) | p-OH | m-OH | CH₃ |
| (68) | o-CH₃ | H | CH₃ |
| (69) | m-CH₃ | H | CH₃ |
| (70) | p-CH₃ | H | CH₃ |
| (71) | o-CH₃ | o-Cl | CH₃ |
| (72) | m-CH₃ | o-Cl | CH₃ |
| (73) | p-CH₃ | o-Cl | CH₃ |
| (74) | m-CH₃ | m-Cl | CH₃ |
| (75) | p-CH₃ | m-Cl | CH₃ |
| (76) | o-CH₃ | o-F | CH₃ |
| (77) | m-CH₃ | o-F | CH₃ |
| (78) | p-CH₃ | o-F | CH₃ |
| (79) | o-CH₃ | m-F | CH₃ |
| (80) | m-CH₃ | m-F | CH₃ |
| (81) | p-CH₃ | m-F | CH₃ |
| (82) | o-CH₃ | p-F | CH₃ |
| (83) | m-CH₃ | p-F | CH₃ |
| (84) | o-CH₃ | o-CH₃ | CH₃ |
| (85) | m-CH₃ | o-CH₃ | CH₃ |
| (86) | p-CH₃ | o-CH₃ | CH₃ |
| (87) | m-CH₃ | m-CH₃ | CH₃ |
| (88) | p-CH₃ | m-CH₃ | CH₃ |
| (89) | o-F | H | C₂H₅ |
| (90) | m-F | H | C₂H₅ |
| (91) | p-F | H | C₂H₅ |
| (92) | o-Cl | H | C₂H₅ |
| (93) | m-Cl | H | C₂H₅ |
| (94) | p-Cl | H | C₂H₅ |
| (95) | o-Cl | o-Cl | C₂H₅ |
| (96) | m-Cl | o-Cl | C₂H₅ |
| (97) | p-Cl | o-Cl | C₂H₅ |
| (98) | m-Cl | m-Cl | C₂H₅ |
| (99) | p-Cl | m-Cl | C₂H₅ |
| (100) | o-Cl | o-F | C₂H₅ |
| (101) | m-Cl | o-F | C₂H₅ |
| (102) | p-Cl | o-F | C₂H₅ |
| (103) | o-Cl | m-F | C₂H₅ |
| (104) | m-Cl | m-F | C₂H₅ |
| (105) | p-Cl | m-F | C₂H₅ |
| (106) | o-Cl | p-F | C₂H₅ |
| (107) | m-Cl | p-F | C₂H₅ |
| (108) | o-F | o-F | C₂H₅ |
| (109) | m-F | o-F | C₂H₅ |
| (110) | p-F | o-F | C₂H₅ |
| (111) | m-F | m-F | C₂H₅ |
| (112) | p-F | m-F | C₂H₅ |
| (113) | o-OCH₃ | H | C₂H₅ |
| (114) | m-OCH₃ | H | C₂H₅ |
| (115) | p-OCH₃ | H | C₂H₅ |
| (116) | o-OCH₃ | o-Cl | C₂H₅ |
| (117) | m-OCH₃ | o-Cl | C₂H₅ |
| (118) | p-OCH₃ | o-Cl | C₂H₅ |
| (119) | m-OCH₃ | m-Cl | C₂H₅ |
| (120) | p-OCH₃ | m-Cl | C₂H₅ |
| (121) | o-OCH₃ | o-F | C₂H₅ |
| (122) | m-OCH₃ | o-F | C₂H₅ |
| (123) | p-OCH₃ | o-F | C₂H₅ |
| (124) | o-OCH₃ | m-F | C₂H₅ |
| (125) | m-OCH₃ | m-F | C₂H₅ |
| (126) | p-OCH₃ | m-F | C₂H₅ |
| (127) | o-OCH₃ | p-F | C₂H₅ |
| (128) | m-OCH₃ | p-F | C₂H₅ |
| (129) | o-OCH₃ | o-OCH₃ | C₂H₅ |
| (130) | m-OCH₃ | o-OCH₃ | C₂H₅ |
| (131) | p-OCH₃ | o-OCH₃ | C₂H₅ |
| (132) | m-OCH₃ | m-OCH₃ | C₂H₅ |
| (133) | p-OCH₃ | m-OCH₃ | C₂H₅ |
| (134) | o-OH | H | C₂H₅ |
| (135) | m-OH | H | C₂H₅ |
| (136) | p-OH | H | C₂H₅ |
| (137) | o-OH | o-Cl | C₂H₅ |
| (138) | m-OH | o-Cl | C₂H₅ |
| (139) | p-OH | o-Cl | C₂H₅ |
| (140) | m-OH | m-Cl | C₂H₅ |
| (141) | p-OH | m-Cl | C₂H₅ |
| (142) | o-OH | o-F | C₂H₅ |
| (143) | m-OH | o-F | C₂H₅ |
| (144) | p-OH | o-F | C₂H₅ |
| (145) | o-OH | m-F | C₂H₅ |
| (146) | m-OH | m-F | C₂H₅ |
| (147) | p-OH | m-F | C₂H₅ |
| (148) | o-OH | p-F | C₂H₅ |
| (149) | m-OH | p-F | C₂H₅ |
| (150) | o-OH | o-OH | C₂H₅ |
| (151) | m-OH | o-OH | C₂H₅ |
| (152) | p-OH | o-OH | C₂H₅ |
| (153) | m-OH | m-OH | C₂H₅ |
| (154) | p-OH | m-OH | C₂H₅ |
| (155) | o-CH₃ | H | C₂H₅ |
| (156) | m-CH₃ | H | C₂H₅ |
| (157) | p-CH₃ | H | C₂H₅ |
| (158) | o-CH₃ | o-Cl | C₂H₅ |
| (159) | m-CH₃ | o-Cl | C₂H₅ |
| (160) | p-CH₃ | o-Cl | C₂H₅ |
| (161) | m-CH₃ | m-Cl | C₂H₅ |
| (162) | p-CH₃ | m-Cl | C₂H₅ |
| (163) | o-CH₃ | o-F | C₂H₅ |
| (164) | m-CH₃ | o-F | C₂H₅ |
| (165) | p-CH₃ | o-F | C₂H₅ |
| (166) | o-CH₃ | m-F | C₂H₅ |
| (167) | m-CH₃ | m-F | C₂H₅ |
| (168) | p-CH₃ | m-F | C₂H₅ |
| (169) | o-CH₃ | p-F | C₂H₅ |
| (170) | m-CH₃ | p-F | C₂H₅ |
| (171) | o-CH₃ | o-CH₃ | C₂H₅ |
| (172) | m-CH₃ | o-CH₃ | C₂H₅ |
| (173) | p-CH₃ | o-CH₃ | C₂H₅ |
| (174) | m-CH₃ | m-CH₃ | C₂H₅ |
| (175) | p-CH₃ | m-CH₃ | C₂H₅ |

Analog werden unter Verwendung der entsprechenden Vorstufen die folgenden Verbindungen der Formel IIIb erhalten:

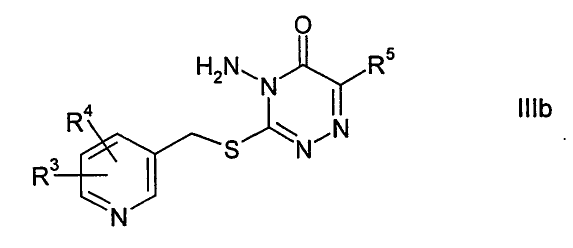

| | R³ | R⁴ | R⁵ |
|---|---|---|---|
| (176) | o-F | H | CH₃ |
| (177) | m-F | H | CH₃ |
| (178) | p-F | H | CH₃ |
| (179) | o-Cl | H | CH₃ |
| (180) | m-Cl | H | CH₃ |
| (181) | p-Cl | H | CH₃ |
| (182) | H | H | CH₃ |
| (183) | o-OCH₃ | H | CH₃ |
| (184) | m-OCH₃ | H | CH₃ |
| (185) | p-OCH₃ | H | CH₃ |

Analog werden unter Verwendung der entsprechenden Vorstufen die folgenden Verbindungen der Formel IIIc erhalten:

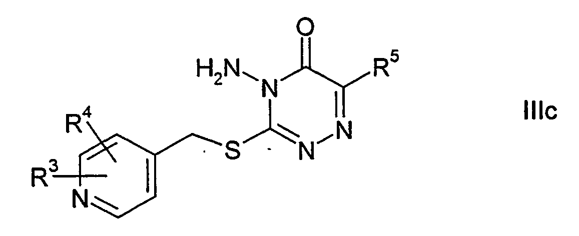

| | R³ | R⁴ | R⁵ |
|---|---|---|---|
| (186) | o-F | H | CH₃ |
| (187) | m-F | H | CH₃ |
| (188) | o-Cl | H | CH₃ |
| (189) | m-Cl | H | CH₃ |
| (190) | H | H | CH₃ |
| (191) | o-OCH₃ | H | CH₃ |
| (192) | m-OCH₃ | H | CH₃ |

### Beispiel 193

Eine Lösung von 0.080 g 4-Amino-3-(2-fluor-benzylsulfanyl)-6-methyl-4*H-*[1,2,4]triazin-5-on in 4 ml Ethanol wurden mit 0.054 g 3-Ethoxy-4-methoxybenzaldehyd und 0.017 ml Essigsäure versetzt und bei 65°C gerührt. Der entstandene Niederschlag wurde abgesaugt, wodurch 4-[(3-Ethoxy-4-methoxy-benzylidene)-amino]-3-(2-fluor-benzylsulfanyl)-6-methyl-4*H-*[1,2,4]triazin-5-on erhalten wurde.

Analog werden unter Verwendung der entsprechenden Ausgangsverbindungen die folgenden Verbindungen der Formel la erhalten:

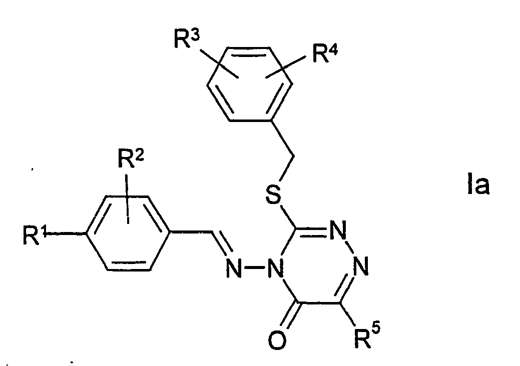

| | R¹ | R² | R³ | R⁴ | R⁵ | |
|---|---|---|---|---|---|---|
| (194) | OCH₃ | m-OC₂H₅ | H | H | CH₃ | |
| (195) | OCH₃ | m-OC₂H₅ | o-Cl | H | CH₃ | (Smp.157°C) |
| (196) | OCH₃ | m-OC₂H₅ | m-Cl | H | CH₃ | |
| (197) | OCH₃ | m-OC₂H₅ | p-Cl | H | CH₃ | |
| (198) | OCH₃ | m-OC₂H₅ | o-F | H | CH₃ | (Smp.155°C) |
| (199) | OCH₃ | m-OC₂H₅ | m-F | H | CH₃ | |
| (200) | OCH₃ | m-OC₂H₅ | p-F | H | CH₃ | |
| (201) | OCH₃ | m-OC₂H₅ | o-Cl | o-Cl | CH₃ | |
| (202) | OCH₃ | m-OC₂H₅ | m-Cl | o-Cl | CH₃ | |
| (203) | OCH₃ | m-OC₂H₅ | p-Cl | o-Cl | CH₃ | |
| (204) | OCH₃ | m-OC₂H₅ | m-Cl | m-Cl | CH₃ | |
| (205) | OCH₃ | m-OC₂H₅ | p-Cl | m-Cl | CH₃ | |
| (206) | OCH₃ | m-OC₂H₅ | o-Cl | o-F | CH₃ | (Smp.234°C) |
| (207) | OCH₃ | m-OC₂H₅ | m-Cl | o-F | CH₃ | |
| (208) | OCH₃ | m-OC₂H₅ | p-Cl | o-F | CH₃ | |
| (209) | OCH₃ | m-OC₂H₅ | o-Cl | m-F | CH₃ | |
| (210) | OCH₃ | m-OC₂H₅ | m-Cl | m-F | CH₃ | |
| (211) | OCH₃ | m-OC₂H₅ | p-Cl | m-F | CH₃ | |
| (212) | OCH₃ | m-OC₂H₅ | o-Cl | p-F | CH₃ | |
| (213) | OCH₃ | m-OC₂H₅ | m-Cl | p-F | CH₃ | |
| (214) | OCH₃ | m-OC₂H₅ | o-F | o-F | CH₃ | |
| (215) | OCH₃ | m-OC₂H₅ | m-F | o-F | CH₃ | |
| (216) | OCH₃ | m-OC₂H₅ | p-F | o-F | CH₃ | |
| (217) | OCH₃ | m-OC₂H₅ | m-F | m-F | CH₃ | |
| (218) | OCH₃ | m-OC₂H₅ | p-F | m-F | CH₃ | |
| (219) | OCH₃ | m-OC₂H₅ | o-OCH₃ | H | CH₃ | |
| (220) | OCH₃ | m-OC₂H₅ | m-OCH₃ | H | CH₃ | |
| (221) | OCH₃ | m-OC₂H₅ | p-OCH₃ | H | CH₃ | |
| (222) | OCH₃ | m-OC₂H₅ | o-OCH₃ | o-Cl | CH₃ | |
| (223) | OCH₃ | m-OC₂H₅ | m-OCH₃ | o-Cl | CH₃ | |
| (224) | OCH₃ | m-OC₂H₅ | p-OCH₃ | o-Cl | CH₃ | |
| (225) | OCH₃ | m-OC₂H₅ | m-OCH₃ | m-Cl | CH₃ | |
| (226) | OCH₃ | m-OC₂H₅ | p-OCH₃ | m-Cl | CH₃ | |
| (227) | OCH₃ | m-OC₂H₅ | o-OCH₃ | o-F | CH₃ | |
| (228) | OCH₃ | m-OC₂H₅ | m-OCH₃ | o-F | CH₃ | |
| (229) | OCH₃ | m-OC₂H₅ | p-OCH₃ | o-F | CH₃ | |
| (230) | OCH₃ | m-OC₂H₅ | o-OCH₃ | m-F | CH₃ | |
| (231) | OCH₃ | m-OC₂H₅ | m-OCH₃ | m-F | CH₃ | |
| (232) | OCH₃ | m-OC₂H₅ | p-OCH₃ | m-F | CH₃ | |
| (233) | OCH₃ | m-OC₂H₅ | o-OCH₃ | p-F | CH₃ | |
| (234) | OCH₃ | m-OC₂H₅ | m-OCH₃ | p-F | CH₃ | |
| (235) | OCH₃ | m-OC₂H₅ | o-OCH₃ | o-OCH₃ | CH₃ | |
| (236) | OCH₃ | m-OC₂H₅ | m-OCH₃ | o-OCH₃ | CH₃ | |
| (237) | OCH₃ | m-OC₂H₅ | p-OCH₃ | o-OCH₃ | CH₃ | |
| (238) | OCH₃ | m-OC₂H₅ | m-OCH₃ | m-OCH₃ | CH₃ | |
| (239) | OCH₃ | m-OC₂H₅ | p-OCH₃ | m-OCH₃ | CH₃ | |
| (240) | OCH₃ | m-OC₂H₅ | o-OH | H | CH₃ | |
| (241) | OCH₃ | m-OC₂H₅ | m-OH | H | CH₃ | |
| (242) | OCH₃ | m-OC₂H₅ | p-OH | H | CH₃ | |
| (243) | OCH₃ | m-OC₂H₅ | o-OH | o-Cl | CH₃ | |
| (244) | OCH₃ | m-OC₂H₅ | m-OH | o-Cl | CH₃ | |
| (245) | OCH₃ | m-OC₂H₅ | p-OH | o-Cl | CH₃ | |
| (246) | OCH₃ | m-OC₂H₅ | m-OH | m-Cl | CH₃ | |
| (247) | OCH₃ | m-OC₂H₅ | p-OH | m-Cl | CH₃ | |
| (248) | OCH₃ | m-OC₂H₅ | o-OH | o-F | CH₃ | |
| (249) | OCH₃ | m-OC₂H₅ | m-OH | o-F | CH₃ | |
| (250) | OCH₃ | m-OC₂H₅ | p-OH | o-F | CH₃ | |
| (251) | OCH₃ | m-OC₂H₅ | o-OH | m-F | CH₃ | |
| (252) | OCH₃ | m-OC₂H₅ | m-OH | m-F | CH₃ | |
| (253) | OCH₃ | m-OC₂H₅ | p-OH | m-F | CH₃ | |
| (254) | OCH₃ | m-OC₂H₅ | o-OH | p-F | CH₃ | |
| (255) | OCH₃ | m-OC₂H₅ | m-OH | p-F | CH₃ | |
| (256) | OCH₃ | m-OC₂H₅ | o-OH | o-OH | CH₃ | |
| (257) | OCH₃ | m-OC₂H₅ | m-OH | o-OH | CH₃ | |
| (258) | OCH₃ | m-OC₂H₅ | p-OH | o-OH | CH₃ | |
| (259) | OCH₃ | m-OC₂H₅ | m-OH | m-OH | CH₃ | |
| (260) | OCH₃ | m-OC₂H₅ | p-OH | m-OH | CH₃ | |
| (261) | OCH₃ | m-OC₂H₅ | o-CH₃ | H | CH₃ | |
| (262) | OCH₃ | m-OC₂H₅ | m-CH₃ | H | CH₃ | |
| (263) | OCH₃ | m-OC₂H₅ | p-CH₃ | H | CH₃ | |
| (264) | OCH₃ | m-OC₂H₅ | o-CH₃ | o-Cl | CH₃ | |
| (265) | OCH₃ | m-OC₂H₅ | m-CH₃ | o-Cl | CH₃ | |
| (266) | OCH₃ | m-OC₂H₅ | p-CH₃ | o-Cl | CH₃ | |
| (267) | OCH₃ | m-OC₂H₅ | m-CH₃ | m-Cl | CH₃ | |
| (268) | OCH₃ | m-OC₂H₅ | p-CH₃ | m-Cl | CH₃ | |
| (269) | OCH₃ | m-OC₂H₅ | o-CH₃ | o-F | CH₃ | |
| (270) | OCH₃ | m-OC₂H₅ | m-CH₃ | o-F | CH₃ | |
| (271) | OCH₃ | m-OC₂H₅ | p-CH₃ | o-F | CH₃ | |
| (272) | OCH₃ | m-OC₂H₅ | o-CH₃ | m-F | CH₃ | |
| (273) | OCH₃ | m-OC₂H₅ | m-CH₃ | m-F | CH₃ | |
| (274) | OCH₃ | m-OC₂H₅ | p-CH₃ | m-F | CH₃ | |
| (275) | OCH₃ | m-OC₂H₅ | o-CH₃ | p-F | CH₃ | |
| (276) | OCH₃ | m-OC₂H₅ | m-CH₃ | p-F | CH₃ | |
| (277) | OCH₃ | m-OC₂H₅ | o-CH₃ | o-CH₃ | CH₃ | |
| (278) | OCH₃ | m-OC₂H₅ | m-CH₃ | o-CH₃ | CH₃ | |
| (279) | OCH₃ | m-OC₂H₅ | p-CH₃ | o-CH₃ | CH₃ | |
| (280) | OCH₃ | m-OC₂H₅ | m-CH₃ | m-CH₃ | CH₃ | |
| (281) | OCH₃ | m-OC₂H₅ | p-CH₃ | m-CH₃ | CH₃ | |
| (282) | OCH₃ | o-OC₂H₅ | H | H | CH₃ | |
| (283) | OCH₃ | o-OC₂H₅ | o-Cl | H | CH₃ | |
| (284) | OCH₃ | o-OC₂H₅ | m-Cl | H | CH₃ | |
| (285) | OCH₃ | o-OC₂H₅ | p-Cl | H | CH₃ | |
| (286) | OCH₃ | o-OC₂H₅ | o-Cl | o-Cl | CH₃ | |
| (287) | OCH₃ | o-OC₂H₅ | m-Cl | o-Cl | CH₃ | |
| (288) | OCH₃ | o-OC₂H₅ | p-Cl | o-Cl | CH₃ | |
| (289) | OCH₃ | o-OC₂H₅ | m-Cl | m-Cl | CH₃ | |
| (290) | OCH₃ | o-OC₂H₅ | p-Cl | m-Cl | CH₃ | |
| (291) | OCH₃ | o-OC₂H₅ | o-Cl | o-F | CH₃ | |
| (292) | OCH₃ | o-OC₂H₅ | m-Cl | o-F | CH₃ | |
| (293) | OCH₃ | o-OC₂H₅ | p-Cl | o-F | CH₃ | |
| (294) | OCH₃ | o-OC₂H₅ | o-Cl | m-F | CH₃ | |
| (295) | OCH₃ | o-OC₂H₅ | m-Cl | m-F | CH₃ | |
| (296) | OCH₃ | o-OC₂H₅ | p-Cl | m-F | CH₃ | |
| (297) | OCH₃ | o-OC₂H₅ | o-Cl | p-F | CH₃ | |
| (298) | OCH₃ | o-OC₂H₅ | m-Cl | p-F | CH₃ | |
| (299) | OCH₃ | o-OC₂H₅ | o-F | o-F | CH₃ | |
| (300) | OCH₃ | o-OC₂H₅ | m-F | o-F | CH₃ | |
| (301) | OCH₃ | o-OC₂H₅ | p-F | o-F | CH₃ | |
| (302) | OCH₃ | o-OC₂H₅ | m-F | m-F | CH₃ | |
| (303) | OCH₃ | o-OC₂H₅ | p-F | m-F | CH₃ | |
| (304) | OCH₃ | o-OC₂H₅ | o-OCH₃ | H | CH₃ | |
| (305) | OCH₃ | o-OC₂H₅ | m-OCH₃ | H | CH₃ | |
| (306) | OCH₃ | o-OC₂H₅ | p-OCH₃ | H | CH₃ | |
| (307) | OCH₃ | o-OC₂H₅ | o-OCH₃ | o-Cl | CH₃ | |
| (308) | OCH₃ | o-OC₂H₅ | m-OCH₃ | o-Cl | CH₃ | |
| (309) | OCH₃ | o-OC₂H₅ | p-OCH₃ | o-Cl | CH₃ | |
| (310) | OCH₃ | o-OC₂H₅ | m-OCH₃ | m-Cl | CH₃ | |
| (311) | OCH₃ | o-OC₂H₅ | p-OCH₃ | m-Cl | CH₃ | |
| (312) | OCH₃ | o-OC₂H₅ | o-OCH₃ | o-F | CH₃ | |
| (313) | OCH₃ | o-OC₂H₅ | m-OCH₃ | o-F | CH₃ | |
| (314) | OCH₃ | o-OC₂H₅ | p-OCH₃ | o-F | CH₃ | |
| (315) | OCH₃ | o-OC₂H₅ | o-OCH₃ | m-F | CH₃ | |
| (316) | OCH₃ | o-OC₂H₅ | m-OCH₃ | m-F | CH₃ | |
| (317) | OCH₃ | o-OC₂H₅ | p-OCH₃ | m-F | CH₃ | |
| (318) | OCH₃ | o-OC₂H₅ | o-OCH₃ | p-F | CH₃ | |
| (319) | OCH₃ | o-OC₂H₅ | m-OCH₃ | p-F | CH₃ | |
| (320) | OCH₃ | o-OC₂H₅ | o-OCH₃ | o-OCH₃ | CH₃ | |
| (321) | OCH₃ | o-OC₂H₅ | m-OCH₃ | o-OCH₃ | CH₃ | |
| (322) | OCH₃ | o-OC₂H₅ | p-OCH₃ | o-OCH₃ | CH₃ | |
| (323) | OCH₃ | o-OC₂H₅ | m-OCH₃ | m-OCH₃ | CH₃ | |
| (324) | OCH₃ | o-OC₂H₅ | p-OCH₃ | m-OCH₃ | CH₃ | |
| (325) | OCH₃ | o-OC₂H₅ | o-OH | H | CH₃ | |
| (326) | OCH₃ | o-OC₂H₅ | m-OH | H | CH₃ | |
| (327) | OCH₃ | o-OC₂H₅ | p-OH | H | CH₃ | |
| (328) | OCH₃ | o-OC₂H₅ | o-OH | o-Cl | CH₃ | |
| (329) | OCH₃ | o-OC₂H₅ | m-OH | o-Cl | CH₃ | |
| (330) | OCH₃ | o-OC₂H₅ | p-OH | o-Cl | CH₃ | |
| (331) | OCH₃ | o-OC₂H₅ | m-OH | m-Cl | CH₃ | |
| (332) | OCH₃ | o-OC₂H₅ | p-OH | m-Cl | CH₃ | |
| (333) | OCH₃ | o-OC₂H₅ | o-OH | o-F | CH₃ | |
| (334) | OCH₃ | o-OC₂H₅ | m-OH | o-F | CH₃ | |
| (335) | OCH₃ | o-OC₂H₅ | p-OH | o-F | CH₃ | |
| (336) | OCH₃ | o-OC₂H₅ | o-OH | m-F | CH₃ | |
| (337) | OCH₃ | o-OC₂H₅ | m-OH | m-F | CH₃ | |
| (338) | OCH₃ | o-OC₂H₅ | p-OH | m-F | CH₃ | |
| (339) | OCH₃ | o-OC₂H₅ | o-OH | p-F | CH₃ | |
| (340) | OCH₃ | o-OC₂H₅ | m-OH | p-F | CH₃ | |
| (341) | OCH₃ | o-OC₂H₅ | o-OH | o-OH | CH₃ | |
| (342) | OCH₃ | o-OC₂H₅ | m-OH | o-OH | CH₃ | |
| (343) | OCH₃ | o-OC₂H₅ | p-OH | o-OH | CH₃ | |
| (344) | OCH₃ | o-OC₂H₅ | m-OH | m-OH | CH₃ | |
| (345) | OCH₃ | o-OC₂H₅ | p-OH | m-OH | CH₃ | |
| (346) | OCH₃ | o-OC₂H₅ | o-CH₃ | H | CH₃ | |
| (347) | OCH₃ | o-OC₂H₅ | m-CH₃ | H | CH₃ | |
| (348) | OCH₃ | o-OC₂H₅ | p-CH₃ | H | CH₃ | |
| (349) | OCH₃ | o-OC₂H₅ | o-CH₃ | o-Cl | CH₃ | |
| (350) | OCH₃ | o-OC₂H₅ | m-CH₃ | o-Cl | CH₃ | |
| (351) | OCH₃ | o-OC₂H₅ | p-CH₃ | o-Cl | CH₃ | |
| (352) | OCH₃ | o-OC₂H₅ | m-CH₃ | m-Cl | CH₃ | |
| (353) | OCH₃ | o-OC₂H₅ | p-CH₃ | m-Cl | CH₃ | |
| (354) | OCH₃ | o-OC₂H₅ | o-CH₃ | o-F | CH₃ | |
| (355) | OCH₃ | o-OC₂H₅ | m-CH₃ | o-F | CH₃ | |
| (356) | OCH₃ | o-OC₂H₅ | p-CH₃ | o-F | CH₃ | |
| (357) | OCH₃ | o-OC₂H₅ | o-CH₃ | m-F | CH₃ | |
| (358) | OCH₃ | o-OC₂H₅ | m-CH₃ | m-F | CH₃ | |
| (359) | OCH₃ | o-OC₂H₅ | p-CH₃ | m-F | CH₃ | |
| (360) | OCH₃ | o-OC₂H₅ | o-CH₃ | p-F | CH₃ | |
| (361) | OCH₃ | o-OC₂H₅ | m-CH₃ | p-F | CH₃ | |
| (362) | OCH₃ | o-OC₂H₅ | o-CH₃ | o-CH₃ | CH₃ | |
| (363) | OCH₃ | o-OC₂H₅ | m-CH₃ | o-CH₃ | CH₃ | |
| (364) | OCH₃ | o-OC₂H₅ | p-CH₃ | o-CH₃ | CH₃ | |
| (365) | OCH₃ | o-OC₂H₅ | m-CH₃ | m-CH₃ | CH₃ | |
| (366) | OCH₃ | o-OC₂H₅ | p-CH₃ | m-CH₃ | CH₃ | |
| (367) | OCH₃ | m-OCH₃ | H | H | CH₃ | |
| (368) | OCH₃ | m-OCH₃ | o-Cl | H | CH₃ | |
| (369) | OCH₃ | m-OCH₃ | m-Cl | H | CH₃ | |
| (370) | OCH₃ | m-OCH₃ | p-Cl | H | CH₃ | |
| (371) | OCH₃ | m-OCH₃ | o-Cl | o-Cl | CH₃ | |
| (372) | OCH₃ | m-OCH₃ | m-Cl | o-Cl | CH₃ | |
| (373) | OCH₃ | m-OCH₃ | p-Cl | o-Cl | CH₃ | |
| (374) | OCH₃ | m-OCH₃ | m-Cl | m-Cl | CH₃ | |
| (375) | OCH₃ | m-OCH₃ | p-Cl | m-Cl | CH₃ | |
| (376) | OCH₃ | m-OCH₃ | o-Cl | o-F | CH₃ | |
| (377) | OCH₃ | m-OCH₃ | m-Cl | o-F | CH₃ | |
| (378) | OCH₃ | m-OCH₃ | p-Cl | o-F | CH₃ | |
| (379) | OCH₃ | m-OCH₃ | o-Cl | m-F | CH₃ | |
| (380) | OCH₃ | m-OCH₃ | m-Cl | m-F | CH₃ | |
| (381) | OCH₃ | m-OCH₃ | p-Cl | m-F | CH₃ | |
| (382) | OCH₃ | m-OCH₃ | o-Cl | p-F | CH₃ | |
| (383) | OCH₃ | m-OCH₃ | m-Cl | p-F | CH₃ | |
| (384) | OCH₃ | m-OCH₃ | o-F | o-F | CH₃ | |
| (385) | OCH₃ | m-OCH₃ | m-F | o-F | CH₃ | |
| (386) | OCH₃ | m-OCH₃ | p-F | o-F | CH₃ | |
| (387) | OCH₃ | m-OCH₃ | m-F | m-F | CH₃ | |
| (388) | OCH₃ | m-OCH₃ | p-F | m-F | CH₃ | |
| (389) | OCH₃ | m-OCH₃ | o-OCH₃ | H | CH₃ | |
| (390) | OCH₃ | m-OCH₃ | m-OCH₃ | H | CH₃ | |
| (391) | OCH₃ | m-OCH₃ | p-OCH₃ | H | CH₃ | |
| (392) | OCH₃ | m-OCH₃ | o-OCH₃ | o-Cl | CH₃ | |
| (393) | OCH₃ | m-OCH₃ | m-OCH₃ | o-Cl | CH₃ | |
| (394) | OCH₃ | m-OCH₃ | p-OCH₃ | o-Cl | CH₃ | |
| (395) | OCH₃ | m-OCH₃ | m-OCH₃ | m-Cl | CH₃ | |
| (396) | OCH₃ | m-OCH₃ | p-OCH₃ | m-Cl | CH₃ | |
| (397) | OCH₃ | m-OCH₃ | o-OCH₃ | o-F | CH₃ | |
| (398) | OCH₃ | m-OCH₃ | m-OCH₃ | o-F | CH₃ | |
| (399) | OCH₃ | m-OCH₃ | p-OCH₃ | o-F | CH₃ | |
| (400) | OCH₃ | m-OCH₃ | o-OCH₃ | m-F | CH₃ | |
| (401) | OCH₃ | m-OCH₃ | m-OCH₃ | m-F | CH₃ | |
| (402) | OCH₃ | m-OCH₃ | p-OCH₃ | m-F | CH₃ | |
| (403) | OCH₃ | m-OCH₃ | o-OCH₃ | p-F | CH₃ | |
| (404) | OCH₃ | m-OCH₃ | m-OCH₃ | p-F | CH₃ | |
| (405) | OCH₃ | m-OCH₃ | o-OCH₃ | o-OCH₃ | CH₃ | |
| (406) | OCH₃ | m-OCH₃ | m-OCH₃ | o-OCH₃ | CH₃ | |
| (407) | OCH₃ | m-OCH₃ | p-OCH₃ | o-OCH₃ | CH₃ | |
| (408) | OCH₃ | m-OCH₃ | m-OCH₃ | m-OCH₃ | CH₃ | |
| (409) | OCH₃ | m-OCH₃ | p-OCH₃ | m-OCH₃ | CH₃ | |
| (410) | OCH₃ | m-OCH₃ | o-OH | H | CH₃ | |
| (411) | OCH₃ | m-OCH₃ | m-OH | H | CH₃ | |
| (412) | OCH₃ | m-OCH₃ | p-OH | H | CH₃ | |
| (413) | OCH₃ | m-OCH₃ | o-OH | o-Cl | CH₃ | |
| (414) | OCH₃ | m-OCH₃ | m-OH | o-Cl | CH₃ | |
| (415) | OCH₃ | m-OCH₃ | p-OH | o-Cl | CH₃ | |
| (416) | OCH₃ | m-CH₃ | m-OH | m-Cl | CH₃ | |
| (417) | OCH₃ | m-CH₃ | p-OH | m-Cl | CH₃ | |
| (418) | OCH₃ | m-CH₃ | o-OH | o-F | CH₃, | |
| (419) | OCH₃ | m-OCH₃ | m-OH | o-F | CH₃ | |
| (420) | OCH₃ | m-OCH₃ | p-OH | o-F | CH₃ | |
| (421) | OCH₃ | m-OCH₃ | o-OH | m-F | CH₃ | |
| (422) | OCH₃ | m-OCH₃ | m-OH | m-F | CH₃ | |
| (423) | OCH₃ | m-OCH₃ | p-OH | m-F | CH₃ | |
| (424) | OCH₃ | m-OCH₃ | o-OH | p-F | CH₃ | |
| (425) | OCH₃ | m-OCH₃ | m-OH | p-F | CH₃ | |
| (426) | OCH₃ | m-OCH₃ | o-OH | o-OH | CH₃ | |
| (427) | OCH₃ | m-OCH₃ | m-OH | o-OH | CH₃ | |
| (428) | OCH₃ | m-OCH₃ | p-OH | o-OH | CH₃ | |
| (429) | OCH₃ | m-OCH₃ | m-OH | m-OH | CH₃ | |
| (430) | OCH₃ | m-OCH₃ | p-OH | m-OH | CH₃ | |
| (431) | OCH₃ | m-OCH₃ | o-CH₃ | H | CH₃ | |
| (432) | OCH₃ | m-OCH₃ | m-CH₃ | H | CH₃ | |
| (433) | OCH₃ | m-CH₃ | p-CH₃ | H | CH₃ | |
| (434) | OCH₃ | m-OCH₃ | o-CH₃ | o-Cl | CH₃ | |
| (435) | OCH₃ | m-OCH₃ | m-CH₃ | o-Cl | CH₃ | |
| (436) | OCH₃ | m-OCH₃ | p-CH₃ | o-Cl | CH₃ | |
| (437) | OCH₃ | m-OCH₃ | m-CH₃ | m-Cl | CH₃ | |
| (438) | OCH₃ | m-OCH₃ | p-CH₃ | m-Cl | CH₃ | |
| (439) | OCH₃ | m-OCH₃ | o-CH₃ | o-F | CH₃ | |
| (440) | OCH₃ | m-OCH₃ | m-CH₃ | o-F | CH₃ | |
| (441) | OCH₃ | m-OCH₃ | p-CH₃ | o-F | CH₃ | |
| (442) | OCH₃ | m-OCH₃ | o-CH₃ | m-F | CH₃ | |
| (443) | OCH₃ | m-OCH₃ | m-CH₃ | m-F | CH₃ | |
| (444) | OCH₃ | m-OCH₃ | p-CH₃ | m-F | CH₃ | |
| (445) | OCH₃ | m-OCH₃ | o-CH₃ | p-F | CH₃ | |
| (446) | OCH₃ | m-OCH₃ | m-CH₃ | p-F | CH₃ | |
| (447) | OCH₃ | m-OCH₃ | o-CH₃ | o-CH₃ | CH₃ | |
| (448) | OCH₃ | m-OCH₃ | m-CH₃ | o-CH₃ | CH₃ | |
| (449) | OCH₃ | m-OCH₃ | p-CH₃ | o-CH₃ | CH₃ | |
| (450) | OCH₃ | m-OCH₃ | m-CH₃ | m-CH₃ | CH₃ | |
| (451) | OCH₃ | m-OCH₃ | p-CH₃ | m-CH₃ | CH₃ | |
| (452) | OCH₃ | H | H | H | CH₃ | |
| (453) | OCH₃ | H | o-Cl | H | CH₃ | |
| (454) | OCH₃ | H | m-Cl | H | CH₃ | |
| (455) | OCH₃ | H | p-Cl | H | CH₃ | |
| (456) | OCH₃ | H | o-Cl | o-Cl | CH₃ | |
| (457) | OCH₃ | H | m-Cl | o-Cl | CH₃ | |
| (458) | OCH₃ | H | p-Cl | o-Cl | CH₃ | |
| (459) | OCH₃ | H | m-Cl | m-Cl | CH₃ | |
| (460) | OCH₃ | H | p-Cl | m-Cl | CH₃ | |
| (461) | OCH₃ | H | o-Cl | o-F | CH₃ | |
| (462) | OCH₃ | H | m-Cl | o-F | CH₃ | |
| (463) | OCH₃ | H | p-Cl | o-F | CH₃ | |
| (464) | OCH₃ | H | o-Cl | m-F | CH₃ | |
| (465) | OCH₃ | H | m-Cl | m-F | CH₃ | |
| (466) | OCH₃ | H | p-Cl | m-F | CH₃ | |
| (467) | OCH₃ | H | o-Cl | p-F | CH₃ | |
| (468) | OCH₃ | H | m-Cl | p-F | CH₃ | |
| (469) | OCH₃ | H | o-F | o-F | CH₃ | |
| (470) | OCH₃ | H | m-F | o-F | CH₃ | |
| (471) | OCH₃ | H | p-F | o-F | CH₃ | |
| (472) | OCH₃ | H | m-F | m-F | CH₃ | |
| (473) | OCH₃ | H | p-F | m-F | CH₃ | |
| (474) | OCH₃ | H | o-OCH₃ | H | CH₃ | |
| (475) | OCH₃ | H | m-OCH₃ | H | CH₃ | |
| (476) | OCH₃ | H | p-OCH₃ | H | CH₃ | |
| (477) | OCH₃ | H | o-OCH₃ | o-Cl | CH₃ | |
| (478) | OCH₃ | H | m-OCH₃ | o-Cl | CH₃ | |
| (479) | OCH₃ | H | p-OCH₃ | o-Cl | CH₃ | |
| (480) | OCH₃ | H | m-OCH₃ | m-Cl | CH₃ | |
| (481) | OCH₃ | H | p-OCH₃ | m-Cl | CH₃ | |
| (482) | OCH₃ | H | o-OCH₃ | o-F | CH₃ | |
| (483) | OCH₃ | H | m-OCH₃ | o-F | CH₃ | |
| (484) | OCH₃ | H | p-OCH₃ | o-F | CH₃ | |
| (485) | OCH₃ | H | o-OCH₃ | m-F | CH₃ | |
| (486) | OCH₃ | H | m-OCH₃ | m-F | CH₃ | |
| (487) | OCH₃ | H | p-OCH₃ | m-F | CH₃ | |
| (488) | OCH₃ | H | o-OCH₃ | p-F | CH₃ | |
| (489) | OCH₃ | H | m-OCH₃ | p-F | CH₃ | |
| (490) | OCH₃ | H | o-OCH₃ | o-OCH₃ | CH₃ | |
| (491) | OCH₃ | H | m-OCH₃ | o-OCH₃ | CH₃ | |
| (492) | OCH₃ | H | p-OCH₃ | o-OCH₃ | CH₃ | |
| (493) | OCH₃ | H | m-OCH₃ | m-OCH₃ | CH₃ | |
| (494) | OCH₃ | H | p-OCH₃ | m-OCH₃ | CH₃ | |
| (495) | OCH₃ | H | o-OH | H | CH₃ | |
| (496) | OCH₃ | H | m-OH | H | CH₃ | |
| (497) | OCH₃ | H | p-OH | H | CH₃ | |
| (498) | OCH₃ | H | o-OH | o-Cl | CH₃ | |
| (499) | OCH₃ | H | m-OH | o-Cl | CH₃ | |
| (500) | OCH₃ | H | p-OH | o-Cl | CH₃ | |
| (501) | OCH₃ | H | m-OH | m-Cl | CH₃ | |
| (502) | OCH₃ | H | p-OH | m-Cl | CH₃ | |
| (503) | OCH₃ | H | o-OH | o-F | CH₃ | |
| (504) | OCH₃ | H | m-OH | o-F | CH₃ | |
| (505) | OCH₃ | H | p-OH | o-F | CH₃ | |
| (506) | OCH₃ | H | o-OH | m-F | CH₃ | |
| (507) | OCH₃ | H | m-OH | m-F | CH₃ | |
| (508) | OCH₃ | H | p-OH | m-F | CH₃ | |
| (509) | OCH₃ | H | o-OH | p-F | CH₃ | |
| (510) | OCH₃ | H | m-OH | p-F | CH₃ | |
| (511) | OCH₃ | H | o-OH | o-OH | CH₃ | |
| (512) | OCH₃ | H | m-OH | o-OH | CH₃ | |
| (513) | OCH₃ | H | p-OH | o-OH | CH₃ | |
| (514) | OCH₃ | H | m-OH | m-OH | CH₃ | |
| (515) | OCH₃ | H | p-OH | m-OH | CH₃ | |
| (516) | OCH₃ | H | o-CH₃ | H | CH₃ | |
| (517) | OCH₃ | H | m-CH₃ | H | CH₃ | |
| (518) | OCH₃ | H | p-CH₃ | H | CH₃ | |
| (519) | OCH₃ | H | o-CH₃ | o-Cl | CH₃ | |
| (520) | OCH₃ | H | m-CH₃ | o-Cl | CH₃ | |
| (521) | OCH₃ | H | p-CH₃ | o-Cl | CH₃ | |
| (522) | OCH₃ | H | m-CH₃ | m-Cl | CH₃ | |
| (523) | OCH₃ | H | p-CH₃ | m-Cl | CH₃ | |
| (524) | OCH₃ | H | o-CH₃ | o-F | CH₃ | |
| (525) | OCH₃ | H | m-CH₃ | o-F | CH₃ | |
| (526) | OCH₃ | H | p-CH₃ | o-F | CH₃ | |
| (527) | OCH₃ | H | o-CH₃ | m-F | CH₃ | |
| (528) | OCH₃ | H | m-CH₃ | m-F | CH₃ | |
| (529) | OCH₃ | H | p-CH₃ | m-F | CH₃ | |
| (530) | OCH₃ | H | o-CH₃ | p-F | CH₃ | |
| (531) | OCH₃ | H | m-CH₃ | p-F | CH₃ | |
| (532) | OCH₃ | H | o-CH₃ | o-CH₃ | CH₃ | |
| (533) | OCH₃ | H | m-CH₃ | o-CH₃ | CH₃ | |
| (534) | OCH₃ | H | p-CH₃ | o-CH₃ | CH₃ | |
| (535) | OCH₃ | H | m-CH₃ | m-CH₃ | CH₃ | |
| (536) | OCH₃ | H | p-CH₃ | m-CH₃ | CH₃ | |
| (537) | OCH₃ | o-Cl | H | H | CH₃ | |
| (538) | OCH₃ | o-Cl | o-Cl | H | CH₃ | |
| (539) | OCH₃ | o-Cl | m-Cl | H | CH₃ | |
| (540) | OCH₃ | o-Cl | p-Cl | H | CH₃ | |
| (541) | OCH₃ | o-Cl | o-Cl | o-Cl | CH₃ | |
| (542) | OCH₃ | o-Cl | m-Cl | o-Cl | CH₃ | |
| (543) | OCH₃ | o-Cl | p-Cl | o-Cl | CH₃ | |
| (544) | OCH₃ | o-Cl | m-Cl | m-Cl | CH₃ | |
| (545) | OCH₃ | o-Cl | p-Cl | m-Cl | CH₃ | |
| (546) | OCH₃ | o-Cl | o-Cl | o-F | CH₃ | |
| (547) | OCH₃ | o-Cl | m-Cl | o-F | CH₃ | |
| (548) | OCH₃ | o-Cl | p-Cl | o-F | CH₃ | |
| (549) | OCH₃ | o-Cl | o-Cl | m-F | CH₃ | |
| (550) | OCH₃ | o-Cl | m-Cl | m-F | CH₃ | |
| (551) | OCH₃ | o-Cl | p-Cl | m-F | CH₃ | |
| (552) | OCH₃ | o-Cl | o-Cl | p-F | CH₃ | |
| (553) | OCH₃ | o-Cl | m-Cl | p-F | CH₃ | |
| (554) | OCH₃ | o-Cl | o-F | o-F | CH₃ | |
| (555) | OCH₃ | o-Cl | m-F | o-F | CH₃ | |
| (556) | OCH₃ | o-Cl | p-F | o-F | CH₃ | |
| (557) | OCH₃ | o-Cl | m-F | m-F | CH₃ | |
| (558) | OCH₃ | o-Cl | p-F | m-F | CH₃ | |
| (559) | OCH₃ | o-Cl | o-OCH₃ | H | CH₃ | |
| (560) | OCH₃ | o-Cl | m-OCH₃ | H | CH₃ | |
| (561) | OCH₃ | o-Cl | p-OCH₃ | H | CH₃ | |
| (562) | OCH₃ | o-Cl | o-OCH₃ | o-Cl | CH₃ | |
| (563) | OCH₃ | o-Cl | m-OCH₃ | o-Cl | CH₃ | |
| (564) | OCH₃ | o-Cl | p-OCH₃ | o-Cl | CH₃ | |
| (565) | OCH₃ | o-Cl | m-OCH₃ | m-Cl | CH₃ | |
| (566) | OCH₃ | o-Cl | p-OCH₃ | m-Cl | CH₃ | |
| (567) | OCH₃ | o-Cl | o-OCH₃ | o-F | CH₃ | |
| (568) | OCH₃ | o-Cl | m-OCH₃ | o-F | CH₃ | |
| (569) | OCH₃ | o-Cl | p-OCH₃ | o-F | CH₃ | |
| (570) | OCH₃ | o-Cl | o-OCH₃ | m-F | CH₃ | |
| (571) | OCH₃ | o-Cl | m-OCH₃ | m-F | CH₃ | |
| (572) | OCH₃ | o-Cl | p-OCH₃ | m-F | CH₃ | |
| (573) | OCH₃ | o-Cl | o-OCH₃ | p-F | CH₃ | |
| (574) | OCH₃ | o-Cl | m-OCH₃ | p-F | CH₃ | |
| (575) | OCH₃ | o-Cl | o-OCH₃ | o-OCH₃ | CH₃ | |
| (576) | OCH₃ | o-Cl | m-OCH₃ | o-OCH₃ | CH₃ | |
| (577) | OCH₃ | o-Cl | p-OCH₃ | o-OCH₃ | CH₃ | |
| (578) | OCH₃ | o-Cl | m-OCH₃ | m-OCH₃ | CH₃ | |
| (579) | OCH₃ | o-Cl | p-OCH₃ | m-OCH₃ | CH₃ | |
| (580) | OCH₃ | o-Cl | o-OH | H | CH₃ | |
| (581) | OCH₃ | o-Cl | m-OH | H | CH₃ | |
| (582) | OCH₃ | o-Cl | p-OH | H | CH₃ | |
| (583) | OCH₃ | o-Cl | o-OH | o-Cl | CH₃ | |
| (584) | OCH₃ | o-Cl | m-CH | o-Cl | CH₃ | |
| (585) | OCH₃ | o-Cl | p-OH | o-Cl | CH₃ | |
| (586) | OCH₃ | o-Cl | m-OH | m-Cl | CH₃ | |
| (587) | OCH₃ | o-Cl | p-OH | m-Cl | CH₃ | |
| (588) | OCH₃ | o-Cl | o-OH | o-F | CH₃ | |
| (589) | OCH₃ | o-Cl | m-OH | o-F | CH₃ | |
| (590) | OCH₃ | o-Cl | p-OH | o-F | CH₃ | |
| (591) | OCH₃ | o-Cl | o-OH | m-F | CH₃ | |
| (592) | OCH₃ | o-Cl | m-OH | m-F | CH₃ | |
| (593) | OCH₃ | o-Cl | p-OH | m-F | CH₃ | |
| (594) | OCH₃ | o-Cl | o-OH | p-F | CH₃ | |
| (595) | OCH₃ | o-Cl | m-OH | p-F | CH₃ | |
| (596) | OCH₃ | o-Cl | o-OH | o-OH | CH₃ | |
| (597) | OCH₃ | o-Cl | m-OH | o-OH | CH₃ | |
| (598) | OCH₃ | o-Cl | p-OH | o-OH | CH₃ | |
| (599) | OCH₃ | o-Cl | m-OH | m-OH | CH₃ | |
| (600) | OCH₃ | o-Cl | p-OH | m-OH | CH₃ | |
| (601) | OCH₃ | o-Cl | o-CH₃ | H | CH₃ | |
| (602) | OCH₃ | o-Cl | m-CH₃ | H | CH₃ | |
| (603) | OCH₃ | o-Cl | p-CH₃ | H | CH₃ | |
| (604) | OCH₃ | o-Cl | o-CH₃ | o-Cl | CH₃ | |
| (605) | OCH₃ | o-Cl | m-CH₃ | o-Cl | CH₃ | |
| (606) | OCH₃ | o-Cl | p-CH₃ | o-Cl | CH₃ | |
| (607) | OCH₃ | o-Cl | m-CH₃ | m-Cl | CH₃ | |
| (608) | OCH₃ | o-Cl | p-CH₃ | m-Cl | CH₃ | |
| (609) | OCH₃ | o-Cl | o-CH₃ | o-F | CH₃ | |
| (610) | OCH₃ | o-Cl | m-CH₃ | o-F | CH₃ | |
| (611) | OCH₃ | o-Cl | p-CH₃ | o-F | CH₃ | |
| (612) | OCH₃ | o-Cl | o-CH₃ | m-F | CH₃ | |
| (613) | OCH₃ | o-Cl | m-CH₃ | m-F | CH₃ | |
| (614) | OCH₃ | o-Cl | p-CH₃ | m-F | CH₃ | |
| (615) | OCH₃ | o-Cl | o-CH₃ | p-F | CH₃ | |
| (616) | OCH₃ | o-Cl | m-CH₃ | p-F | CH₃ | |
| (617) | OCH₃ | o-Cl | o-CH₃ | o-CH₃ | CH₃ | |
| (618) | OCH₃ | o-Cl | m-CH₃ | o-CH₃ | CH₃ | |
| (619) | OCH₃ | o-Cl | p-CH₃ | o-CH₃ | CH₃ | |
| (620) | OCH₃ | o-Cl | m-CH₃ | -m-CH₃ | CH₃ | |
| (621) | OCH₃ | o-Cl | p-CH₃ | m-CH₃ | CH₃ | |
| (622) | OCH₃ | m-OC₂H₅ | H | H | C₂H₅ | |
| (623) | OCH₃ | m-OC₂H₅ | o-Cl | H | C₂H₅ | |
| (624) | OCH₃ | m-OC₂H₅ | m-Cl | H | C₂H₅ | |
| (625) | OCH₃ | m-OC₂H₅ | p-Cl | H | C₂H₅ | |
| (626) | OCH₃ | m-OC₂H₅ | o-Cl | o-Cl | C₂H₅ | |
| (627) | OCH₃ | m-OC₂H₅ | m-Cl | o-Cl | C₂H₅ | |
| (628) | OCH₃ | m-OC₂H₅ | p-CI | o-Cl | C₂H₅ | |
| (629) | OCH₃ | m-OC₂H₅ | m-Cl | m-Cl | C₂H₅ | |
| (630) | OCH₃ | m-OC₂H₅ | p-Cl | m-Cl | C₂H₅ | |
| (631) | OCH₃ | m-OC₂H₅ | o-Cl | o-F | C₂H₅ | |
| (632) | OCH₃ | m-OC₂H₅ | m-Cl | o-F | C₂H₅ | |
| (633) | OCH₃ | m-OC₂H₅ | p-Cl | o-F | C₂H₅ | |
| (634) | OCH₃ | m-OC₂H₅ | o-Cl | m-F | C₂H₅ | |
| (635) | OCH₃ | m-OC₂H₅ | m-Cl | m-F | C₂H₅ | |
| (636) | OCH₃ | m-OC₂H₅ | p-Cl | m-F | C₂H₅ | |
| (637) | OCH₃ | m-OC₂H₅ | o-Cl | p-F | C₂H₅ | |
| (638) | OCH₃ | m-OC₂H₅ | m-Cl | p-F | C₂H₅ | |
| (639) | OCH₃ | m-OC₂H₅ | o-F | o-F | C₂H₅ | |
| (640) | OCH₃ | m-OC₂H₅ | m-F | o-F | C₂H₅ | |
| (641) | OCH₃ | m-OC₂H₅ | p-F | o-F | C₂H₅ | |
| (642) | OCH₃ | m-OC₂H₅ | m-F | m-F | C₂H₅ | |
| (643) | OCH₃ | m-OC₂H₅ | p-F | m-F | C₂H₅ | |
| (644) | OCH₃ | m-OC₂H₅ | o-OCH₃ | H | C₂H₅ | |
| (645) | OCH₃ | m-OC₂H₅ | m-OCH₃ | H | C₂H₅ | |
| (646) | OCH₃ | m-OC₂H₅ | p-OCH₃ | H | C₂H₅ | |
| (647) | OCH₃ | m-OC₂H₅ | o-OCH₃ | o-Cl | C₂H₅ | |
| (648) | OCH₃ | m-OC₂H₅ | m-OCH₃ | o-Cl | C₂H₅ | |
| (649) | OCH₃ | m-OC₂H₅ | p-OCH₃ | o-Cl | C₂H₅ | |
| (650) | OCH₃ | m-OC₂H₅ | m-OCH₃ | m-Cl | C₂H₅ | |
| (651) | OCH₃ | m-OC₂H₅ | p-OCH₃ | m-Cl | C₂H₅ | |
| (652) | OCH₃ | m-OC₂H₅ | o-OCH₃ | o-F | C₂H₅ | |
| (653) | OCH₃ | m-OC₂H₅ | m-OCH₃ | o-F | C₂H₅ | |
| (654) | OCH₃ | m-OC₂H₅ | p-OCH₃ | o-F | C₂H₅ | |
| (655) | OCH₃ | m-OC₂H₅ | o-OCH₃ | m-F | C₂H₅ | |
| (656) | OCH₃ | m-OC₂H₅ | m-OCH₃ | m-F | C₂H₅ | |
| (657) | OCH₃ | m-OC₂H₅ | H | H | H | |
| (658) | OCH₃ | m-OC₂H₅ | o-Cl | H | H | |
| (659) | OCH₃ | m-OC₂H₅ | m-Cl | H | H | |
| (660) | OCH₃ | m-OC₂H₅ | p-Cl | H | H | |
| (661) | OCH₃ | m-OC₂H₅ | o-Cl | o-Cl | H | |
| (662) | OCH₃ | m-OC₂H₅ | m-Cl | o-Cl | H | |
| (663) | OCH₃ | m-OC₂H₅ | p-Cl | o-Cl | H | |
| (664) | OCH₃ | m-OC₂H₅ | m-Cl | m-Cl | H | |
| (665) | OCH₃ | m-OC₂H₅ | p-Cl | m-Cl | H | |
| (666) | OCH₃ | m-OC₂H₅ | o-Cl | o-F | H | |
| (667) | OCH₃ | m-OC₂H₅ | m-Cl | o-F | H | |
| (668) | OCH₃ | m-OC₂H₅ | p-Cl | o-F | H | |
| (669) | OCH₃ | 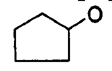 | H | H | CH₃ | |
| (670) | OCH₃ | 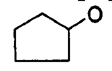 | o-Cl | H | CH₃ | |
| (671) | OCH₃ | 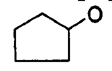 | m-Cl | H | CH₃ | |
| (672) | OCH₃ | 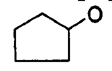 | p-Cl | H | CH₃ | |
| (673) | OCH₃ | 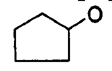 | o-F | H | CH₃ | (Smp.155°C) |
| (674) | OCH₃ | 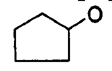 | m-F | H | CH₃ | |
| (675) | OCH₃ | 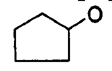 | p-F | H | CH₃ | |
| (676) | OCH₃ | 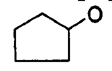 | o-Cl | o-Cl | CH₃ | |
| (677) | OCH₃ | 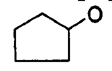 | m-Cl | o-Cl | CH₃ | |
| (678) | OCH₃ | 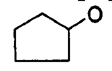 | p-Cl | o-Cl | CH₃ | |
| (679) | OCH₃ | 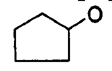 | m-Cl | m-Cl | CH₃ | |
| (680) | OCH₃ | 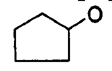 | p-Cl | m-Cl | CH₃ | |
| (681) | OCH₃ | 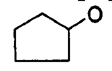 | o-Cl | o-F | CH₃ | (Smp. 180°C) |
| (682) | OCH₃ | 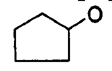 | m-Cl | o-F | CH₃ | |
| (683) | OCH₃ | 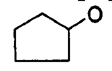 | p-Cl | o-F | CH₃ | |
| (684) | OCH₃ | 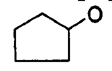 | o-Cl | m-F | CH₃ | |
| (685) | OCH₃ | 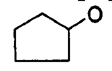 | m-Cl | m-F | CH₃ | |
| (686) | OCH₃ | 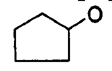 | p-Cl | m-F | CH₃ | |
| (687) | OCH₃ | 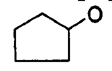 | o-Cl | p-F | CH₃ | |
| (688) | OCH₃ | 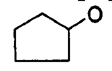 | m-Cl | p-F | CH₃ | |
| (689) | OCH₃ | 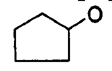 | o-F | o-F | CH₃ | |
| (690) | OCH₃ | 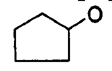 | m-F | o-F | CH₃ | |
| (691) | OCH₃ | 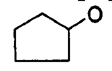 | p-F | o-F | CH₃ | |
| (692) | OCH₃ | 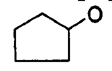 | m-F | m-F | CH₃ | |
| (693) | OCH₃ | 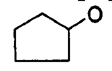 | p-F | m-F | CH₃ | |
| (694) | OCH₃ | 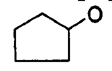 | o-OCH₃ | H | CH₃ | |
| (695) | OCH₃ | 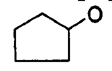 | m-OCH₃ | H | CH₃ | |
| (696) | OCH₃ | 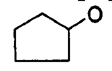 | p-OCH₃ | H | CH₃ | |
| (697) | OCH₃ | 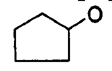 | o-OCH₃ | o-Cl | CH₃ | |
| (698) | OCH₃ | 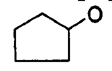 | m-OCH₃ | o-Cl | CH₃ | |
| (699) | OCH₃ | 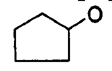 | p-OCH₃ | o-Cl | CH₃ | |
| (700) | OCH₃ | 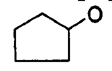 | m-OCH₃ | m-Cl | CH₃ | |
| (701) | OCH₃ | 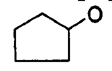 | p-OCH₃ | m-Cl | CH₃ | |
| (702) | OCH₃ | 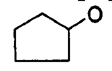 | o-OCH₃ | o-F | CH₃ | |
| (703) | OCH₃ | 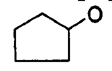 | m-OCH₃ | o-F | CH₃ | |
| (704) | OCH₃ | 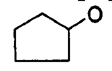 | p-OCH₃ | o-F | CH₃ | |
| (705) | OCH₃ | 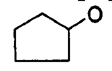 | o-OCH₃ | m-F | CH₃ | |
| (706) | OCH₃ | 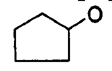 | m-OCH₃ | m-F | CH₃ | |
| (707) | OCH₃ | 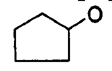 | p-OCH₃ | m-F | CH₃ | |
| (708) | OCH₃ | 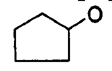 | o-OCH₃ | p-F | CH₃ | |
| (709) | OCH₃ | 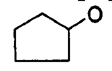 | m-OCH₃ | p-F | CH₃ | |
| (710) | OCH₃ | 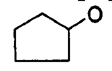 | o-OCH₃ | o-OCH₃ | CH₃ | |
| (711) | OCH₃ | 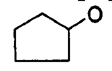 | m-OCH₃ | o-OCH₃ | CH₃ | |
| (712) | OCH₃ | 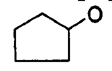 | p-OCH₃ | o-OCH₃ | CH₃ | |
| (713) | OCH₃ | 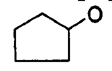 | m-OCH₃ | m-OCH₃ | CH₃ | |
| (714) | OCH₃ | 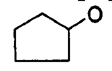 | p-OCH₃ | m-OCH₃ | CH₃ | |
| (715) | OCH₃ | 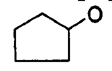 | o-OH | H | CH₃ | |
| (716) | OCH₃ | 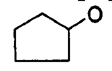 | m-OH | H | CH₃ | |
| (717) | OCH₃ | 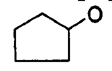 | p-OH | H | CH₃ | |
| (718) | OCH₃ | 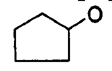 | o-OH | o-Cl | CH₃ | |
| (719) | OCH₃ | 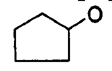 | m-OH | o-Cl | CH₃ | |
| (720) | OCH₃ | 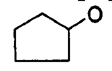 | p-OH | o-Cl | CH₃ | |
| (721) | OCH₃ | 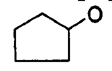 | m-OH | m-Cl | CH₃ | |
| (722) | OCH₃ | 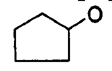 | p-OH | m-Cl | CH₃ | |
| (723) | OCH₃ | 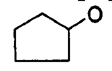 | o-OH | o-F | CH₃ | |
| (724) | OCH₃ | 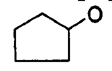 | m-OH | o-F | CH₃ | |
| (725) | OCH₃ | 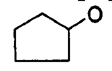 | p-OH | o-F | CH₃ | |
| (726) | OCH₃ | 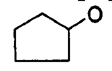 | o-OH | m-F | CH₃ | |
| (727) | OCH₃ | 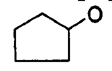 | m-OH | m-F | CH₃ | |
| (728) | OCH₃ | 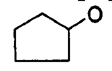 | p-OH | m-F | CH₃ | |
| (729) | OCH₃ | 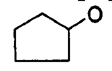 | o-OH | p-F | CH₃ | |
| (730) | OCH₃ | 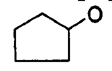 | m-OH | p-F | CH₃ | |
| (731) | OCH₃ | 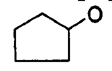 | o-OH | o-OH | CH₃ | |
| (732) | OCH₃ | 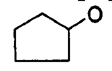 | m-OH | o-OH | CH₃ | |
| (733) | OCH₃ | 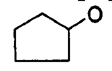 | p-OH | o-OH | CH₃ | |
| (734) | OCH₃ | 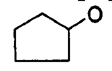 | m-OH | m-OH | CH₃ | |
| (735) | OCH₃ | 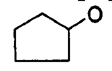 | p-OH | m-OH | CH₃ | |
| (736) | OCH₃ | 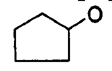 | o-CH₃ | H | CH₃ | |
| (737) | OCH₃ | 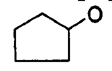 | m-CH₃ | H | CH₃ | |
| (738) | OCH₃ | 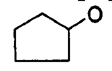 | p-CH₃ | H | CH₃ | |
| (739) | OCH₃ | 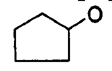 | o-CH₃ | o-Cl | CH₃ | |
| (740) | OCH₃ | 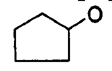 | m-CH₃ | o-Cl | CH₃ | |
| (741) | OCH₃ | 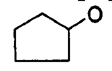 | p-CH₃ | o-Cl | CH₃ | |
| (742) | OCH₃ | 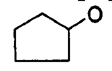 | m-CH₃ | m-Cl | CH₃ | |
| (743) | OCH₃ | 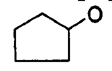 | p-CH₃ | m-Cl | CH₃ | |
| (744) | OCH₃ | 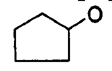 | o-CH₃ | o-F | CH₃ | |
| (745) | OCH₃ | 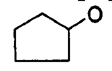 | m-CH₃ | o-F | CH₃ | |
| (746) | OCH₃ | 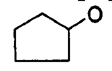 | p-CH₃ | o-F | CH₃ | , |
| (747) | OCH₃ | 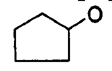 | o-CH₃ | m-F | CH₃ | |
| (748) | OCH₃ | 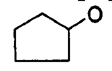 | m-CH₃ | m-F | CH₃ | |
| (749) | OCH₃ | 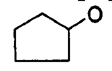 | p-CH₃ | m-F | CH₃ | |
| (750) | OCH₃ | 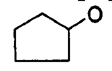 | o-CH₃ | p-F | CH₃ | |
| (751) | OCH₃ | 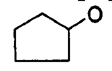 | m-CH₃ | p-F | CH₃ | |
| (752) | OCH₃ | 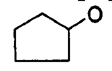 | o-CH₃ | o-CH₃ | CH₃ | |
| (753) | OCH₃ | 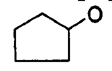 | m-CH₃ | o-CH₃ | CH₃ | |
| (754) | OCH₃ | 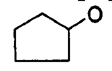 | p-CH₃ | o-CH₃ | CH₃ | |
| (755) | OCH₃ | 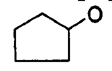 | m-CH₃ | m-CH₃ | CH₃ | |
| (756) | OCH₃ | 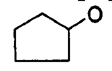 | p-CH₃ | m-CH₃ | CH₃ | |

Analog werden unter Verwendung der entsprechenden Ausgangsverbindungen die folgenden Verbindungen der Formel Ib erhalten:

| 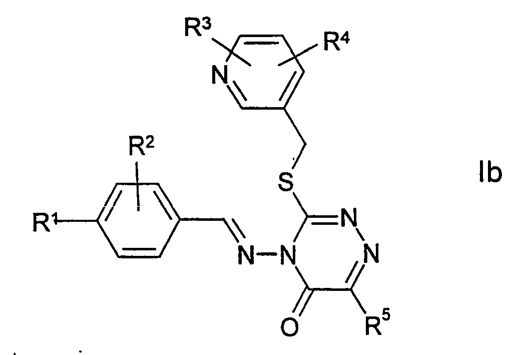 | | | | | | |
|---|---|---|---|---|---|---|
| | R¹ | R² | R³ | R⁴ | R⁵ | |
| (757) | OCH₃ | m-OC₂H₅ | H | H | CH₃ | (Smp.220°C) |
| (758) | OCH₃ | m-OC₂H₅ | o-Cl | H | CH₃ | |
| (759) | OCH₃ | m-OC₂H₅ | m-Cl | H | CH₃ | |
| (760) | OCH₃ | m-OC₂H₅ | p-Cl | H | CH₃ | |
| (761) | OCH₃ | m-OC₂H₅ | o-F | H | CH₃ | |
| (762) | OCH₃ | m-OC₂H₅ | m-F | H | CH₃ | |
| (763) | OCH₃ | m-OC₂H₅ | p-F | H | CH₃ | |
| (764) | OCH₃ | 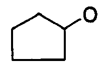 | H | H | CH₃ | (Smp.169°C) |
| (765) | OCH₃ | 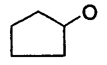 | o-Cl | H | CH₃ | |
| (766) | OCH₃ | 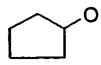 | m-Cl | H | CH₃ | |
| (767) | OCH₃ | 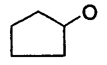 | p-Cl | H | CH₃ | |
| (768) | OCH₃ | 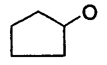 | o-F | H | CH₃ | |
| (769) | OCH₃ | 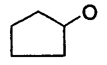 | m-F | H | CH₃ | |
| (770) | OCH₃ | 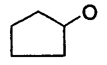 | p-F | H | CH₃ | |

Analog werden unter Verwendung der entsprechenden Ausgangsverbindungen die folgenden Verbindungen der Formel Ic erhalten:

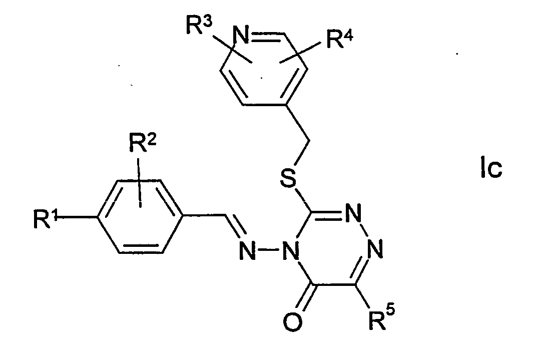

| | R¹ | R² | R³ | R⁴ - | R⁵ |
|---|---|---|---|---|---|
| (771) | OCH₃ | m-OC₂H₅ | H | H | CH₃ |
| (772) | OCH₃ | m-OC₂H₅ | o-Cl | H | CH₃ |
| (773) | OCH₃ | m-OC₂H₅ | m-Cl | H | CH₃ |
| (774) | OCH₃ | m-OC₂H₅ | o-F | H | CH₃ |
| (775) | OCH₃ | m-OC₂H₅ | m-F | H | CH₃ |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 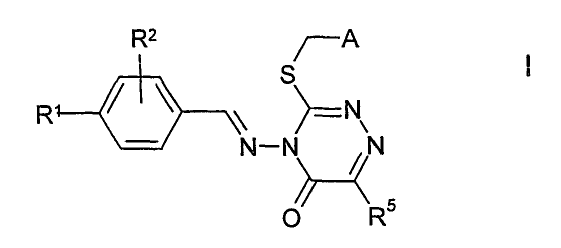 worin
R¹, R² jeweils unabhängig voneinander H, OH, OR⁶, SR⁶, SOR⁶, SO₂R⁶, Hal oder zusammen auch -O-CH₂-O-,
A durch R³ und R⁴ substituiertes Phenyl, 2-, 3- oder 4-Pyridyl, 4- oder 5-Pyrimidyl, 3- oder 4-Pyridazyl, 2- oder 3- Pyrazinyl,
R³, R⁴ jeweils unabhängig voneinander H, OH, OR⁶, SR⁶, SOR⁶, SO₂R⁶, R⁶, Hal oder zusammen auch -O-CH₂-O-,
R⁵ H oder Alkyl mit 1 bis 10 C-Atomen,
R⁶ Alkyl mit 1 bis 10 C-Atomen, das durch 1 bis 5 F- und/oder Cl-Atome substituiert sein kann, Cycloalkyl mit 3-7 C-Atomen, Alkylencycloalkyl mit 5-10 C-Atomen oder Alkenyl mit 2-8 C-Atomen,
Hal F, Cl, Br oder I
bedeuten,
sowie deren physiologisch unbedenklichen Salze und Solvate.

2. Verbindungen der Formel I nach Anspuch 1, worin R⁵ die Bedeutung Methyl aufweist.

3. Verbindungen der Formel I nach einem oder mehreren der Anspüche 1 und 2, worin R¹ und R² jeweils unabhängig voneinander OR⁶ bedeuten.

4. Verbindungen der Formel I nach einem oder mehreren der Anspüche 1 bis 3, worin R⁶ Alkyl mit 1-10 C-Atomen oder Cycloalkyl mit 3-7 C-Atomen bedeutet.

5. Verbindungen der Formel I nach einem oder mehreren der Anspüche 1 bis 4, worin A Phenyl, 2-, 3- oder 4-Pyridyl, 4- oder 5-Pyrimidyl und R³ und R⁴ jeweils unabhängig voneinander R⁶, H, Cl, F, CF₃ oder OR⁶ bedeuten.

6. Verbindungen der Formel (a) bis (h) gemäß Anspruch 1:
(a) 4-[(3-Ethoxy-4-methoxy-benzyliden)-amino]-3-(benzylsulfanyl)-6-methyl-4*H*-[1,2,4]triazin-5-on
(b) 4-[(3-Ethoxy-4-methoxy-benzyliden)-amino]-3-(2-fluorbenzylsulfanyl)-6-methyl-4*H*-[1,2,4]triazin-5-on
(c) 4-[(3-Ethoxy-4-methoxy-benzyliden)-amino]-3-(2-chlor-6-fluorbenzylsulfanyl)-6-methyl-4*H*-[1,2,4]triazin-5-on
(d) 4-[(3-Ethoxy-4-methoxy-benzyliden)-amino]-6-methyl-3-(pyridin-3-ylmethylsulfanyl)-4*H*-[1,2,4]triazin-5-on
(e) 4-[(3-Cyclopentyloxy-4-methoxy-benzyliden)-amino]-3-(benzylsulfanyl)-6-methyl-4*H*-[1,2,4]triazin-5-on
(f) 4-[(3-Cyclopentyloxy-4-methoxy-benzyliden)-amino]-3-(2-fluorbenzylsulfanyl)-6-methyl-4*H*-[1,2,4]triazin-5-on
(g) 4-[(3-Cyclopentyloxy-4-methoxy-benzyliden)-amino]-3-(2-chlor6-fluor-benzylsulfanyl)-6-methyl-4*H*-[1,2,4]triazin-5-on
(h) 4-[(3-Cyclopentyloxy-4-methoxy-benzyliden)-amino]-6-methyl-3-(pyridin-3-ylmethylsulfanyl)-4*H*-[1,2,4]triazin-5-on
sowie deren physiologisch unbedenklichen Salze und Solvate.

7. Verfahren zur Herstellung von Verbindungen der Formel I nach Anspruch 1 sowie deren Salze und Solvate, **dadurch gekennzeichnet, daß** man eine Verbindung der Formel 11 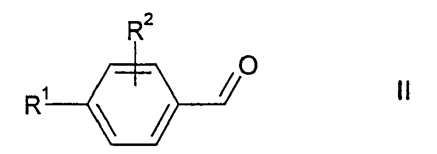 worin
R¹ und R² die angegebenen Bedeutungen haben,
mit einer Verbindung der Formel III 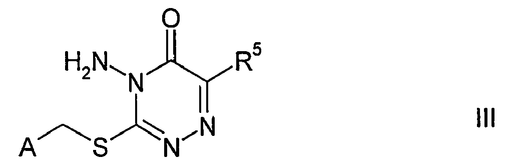 worin
A und R⁵ die in Anspruch 1 angegebenen Bedeutungen haben,
umsetzt,
und/oder daß man eine basische Verbindung der Formel I durch Behandeln mit einer Säure in eines ihrer Salze überführt.

8. Verbindungen der Formel III 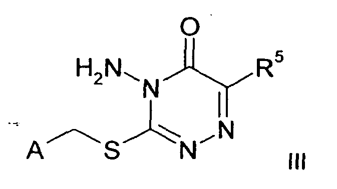 , worin A und R⁵ wie in Anspruch 1 definiert sind, mit der Vorgabe, dass Verbindungen der Formel III, in denen A Phenyl und R³ und R⁴ beide H sind, oder einer von beiden Cl ist, oder A 2-Pyridyl ist, ausgeschlossen sind.

9. Verbindungen der Formel I nach einem oder mehreren der Anspüche 1 bis 6 und ihre physiologisch unbedenklichen Salze und Solvate als Arzneimittel.

10. Verwendung der Verbindungen der Formel I nach einem oder mehreren der Ansprüche 1 bis 6 und/oder ihre physiologisch unbedenklichen Salze und/oder Solvate zur Herstellung eines Arzneimittels.

11. Verbindungen der Formel I nach einem oder mehreren der Anspüche 1 bis 6 und ihre physiologisch unbedenklichen Salze und Solvate als Phosphodiesterase IV-Hemmer.

12. Pharmazeutische Zubereitung, **gekennzeichnet durch** einen Gehalt an mindestens einer Verbindung der Formel I nach einem oder mehreren der Anspüche 1 bis 6 und/oder einem ihrer physiologisch unbedenklichen Salze und/oder eines ihrer Solvate.

13. Verfahren zur Herstellung pharmazeutischer Zubereitungen, **dadurch gekennzeichnet, daß** man eine Verbindung der Formel I nach einem oder mehreren der Anspüche 1 bis 6 und/oder eines ihrer physiologischen unbedenklichen Salze und/oder eines ihrer Solvate zusammen mit mindestens einem festen, flüssigen oder halbflüssigen Träger- oder Hilfsstoff in eine geeignete Dosierungsform bringt.

14. Verwendung der Verbindungen der Formel I nach einem oder mehreren der Ansprüche 1 bis 6 und/oder ihre physiologisch unbedenklichen Salze und/oder Solvate zur Herstellung eines Arzneimittels zur Behandlung und Prophylaxe von Herzkrankheiten (myocardial diseases).

15. Verwendung von Verbindungen der Formel I nach einem oder mehreren der Ansprüche 1 bis 6 und/oder deren Salze und/oder Solvate zur Herstellung eines Medikaments zur Behandlung von Herzmuskelerkrankungen mit entzündlichen und immunologischen Charakteristika.

16. Verwendung der Verbindungen der Formel I nach einem oder mehreren der Ansprüche 1 bis 6 und/oder deren Salze und/oder Solvate zur Herstellung eines Medikaments zur Behandlung von koronarer Herzkrankheit, reversibler oder irreversibler Myokardischämie/Reperfusionsschäden, akutem oder chronischem Herzversagen und Restenose einschließlich Instent-Restenose und Stent-in-Stent-Restenose.

17. Verwendung der Verbindungen der Formel I nach einem oder mehreren der Ansprüche 1 bis 6 und/oder ihre physiologisch unbedenklichen Salze und/oder Solvate zur Herstellung eines Arzneimittels zur Prophylaxe und Behandlung von ventrikulärer Umbildung (venticular remodeling) nach Infarkt oder dekompensierter Herzinsuffizienz (congestive heart failure, CHF) unterschiedlicher Ausprägung.

18. Verwendung der Verbindungen der Formel I nach einem oder mehreren der Ansprüche 1 bis 6 und/oder ihre physiologisch unbedenklichen Salze und/oder Solvate zur Herstellung eines Arzneimittels zur Behandlung und Prophylaxe von Krankheiten, die durch einen zu geringen cAMP-Spiegel verusacht werden und/oder durch eine Erhöhung des cAMP-Spiegels beeinflußt werden können.

19. Verwendung der Verbindungen der Formel I nach einem oder mehreren der Ansprüche 1 bis 6 und/oder ihre physiologisch unbedenklichen Salze und/oder Solvate zur Herstellung eines Arzneimittels zur Behandlung und Prophylaxe von Krankheiten, die durch eine Verringerung der Produktion von Tumor Nekrose Faktor (TNF) beeinflußt werden können.

20. Verwendung der Verbindungen der Formel I nach einem oder mehreren der Ansprüche 1 bis 6 und/oder ihre physiologisch unbedenklichen Salze und/oder Solvate zur Herstellung eines Arzneimittels zur Behandlung und Prophylaxe von Krankheiten, die durch eine Überproduktion von Makrophagen und T-Zellen verusacht werden und/oder durch eine Verrringerung der Makrophagen- und T-Zell-Produktion beeinflußt werden können.

21. Verwendung der Verbindungen der Formel I nach einem oder mehreren der Ansprüche 1 bis 6 und/oder ihre physiologisch unbedenklichen Salze und/oder Solvate zur Herstellung eines Arzneimittels zur Behandlung und Prophylaxe von Krankheiten, die durch übermäßige Proliferation der T-Zellen verusacht werden und/oder durch.eine Hemmung der Proliferation der T-Zellen beeinflußt werden können.

22. Verwendung der Verbindungen der Formel I nach einem oder mehreren der Ansprüche 1 bis 6 und/oder deren physiologisch verträglichen Salze und/oder Solvate, zur Herstellung eines Medikamentes zur Behandlung oder Prophylaxe von Krankheiten, die durch Störungen der Regulation der Aktivierung und Degranulation von humanen Eosinophilen hervorgerufen werden.

23. Verbindungen der Formel I nach einem oder mehreren der Anspüche 1 bis 6 und/oder ihre physiologisch unbedenklichen Salze und/oder Solvate zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

24. Verwendung von Verbindungen der Formel I nach einem oder mehreren der Anspüche 1 bis 6 und/oder ihre physiologisch unbedenklichen Salze und/oder Solvate zur Herstellung eines Arzneimittels zur Bekämpfung von allergischen Krankheiten, Asthma, chronischer Bronchitis, atopischer Dermatitis, Psoriasis und anderer Hautkrankheiten, entzündlichen Krankheiten, Autoimmunerkrankungen, wie z.B. rheumatoide Arthritis, multiple Sklerose, Morbus Crohn, Diabetes mellitus oder ulzerative Kolitis, Osteoporose, Transplantatabstoßungsreaktionen, Kachexie, Tumorwachstum oder Tumormetastasen, Sepsis, Gedächtnisstörungen, Atherosklerose und AIDS.

25. Verwendung von Verbindungen der Formel III 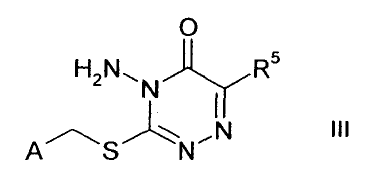 , worin A und R⁵ wie in Anspruch 1 definiert sind, zur Herstellung von Verbindungen der Formel I nach Anspruch 1.

## Claims

1. Compounds of the formula I 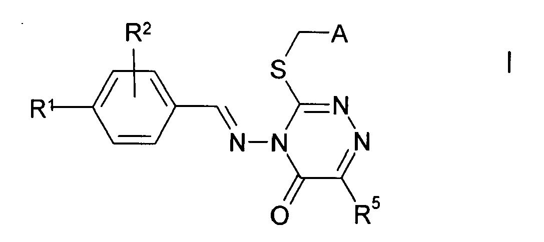 in which
R¹, R² each, independently of one another, denote H, OH, OR⁶, SR⁶, SOR⁶, SO₂R⁶, Hal or together also denote -O-CH₂-O-,
A denotes R³- and R⁴-substituted phenyl, 2-, 3- or 4-pyridyl, 4- or 5-pyrimidyl, 3- or 4-pyridazyl, 2- or 3-pyrazinyl,
R³, R⁴ each, independently of one another, denote H, OH, OR⁶, SR⁶, SOR⁶, SO₂R⁶, R⁶, Hal or together also denote -O-CH₂-O-,
R⁵ denotes H or alkyl having 1 to 10 C atoms,
R⁶ denotes alkyl having 1 to 10 C atoms, which may be substituted by 1 to 5 F and/or Cl atoms,
cycloalkyl having 3-7 C atoms, alkylenecycloalkyl having 5-10 C atoms or alkenyl having 2-8 C atoms,
Hal denotes F, Cl, Br or I,
and physiologically acceptable salts and solvates thereof.

2. Compounds of the formula I according to Claim 1 in which R⁵ has the meaning methyl.

3. Compounds of the formula I according to one or more of Claims 1 and 2 in which R¹ and R² each, independently of one another, denote OR⁶.

4. Compounds of the formula I according to one or more of Claims 1 to 3 in which R⁶ denotes alkyl having 1-10 C atoms or cycloalkyl having 3-7 C atoms.

5. Compounds of the formula I according to one or more of Claims 1 to 4, in which A denotes phenyl, 2-, 3- or 4-pyridyl, 4- or 5-pyrimidyl and R³ and R⁴ each, independently of one another, denote R⁶, H, Cl, F, CF₃ or OR⁶.

6. Compounds of the formulae (a) to (h) according to Claim 1:
(a) 4-[(3-ethoxy-4-methoxybenzylidene)amino]-3-(benzylsulfanyl)-6-methyl-4*H*-[1,2,4]triazin-5-one
(b) 4-[(3-ethoxy-4-methoxybenzylidene)amino]-3-(2-fluorobenzylsulfanyl)-6-methyl-4*H*-[1,2,4]triazin-5-one
(c) 4-[(3-ethoxy-4-methoxybenzylidene)amino]-3-(2-chloro-6-fluorobenzylsulfanyl)-6-methyl-4*H*-[1,2,4]triazin-5-one
(d) 4-[(3-ethoxy-4-methoxybenzylidene)amino]-6-methyl-3-(pyridin-3-ylmethylsulfanyl)-4*H*-[1,2,4]triazin-5-one
(e) 4-[(3-cyclopentyloxy-4-methoxybenzylidene)amino]-3-(benzylsulfanyl)-6-methyl-4*H*-[1,2,4]triazin-5-one
(f) 4-[(3-cyclopentyloxy-4-methoxybenzylidene)amino]-3-(2-fluorobenzylsulfanyl)-6-methyl-4*H*-[1,2,4]triazin-5-one
(g) 4-[(3-cyclopentyloxy-4-methoxybenzylidene)amino]-3-(2-chloro-6-fluorobenzylsulfanyl)-6-methyl-4*H*-[1,2,4]triazin-5-one
(h) 4-[(3-cyclopentyloxy-4-methoxybenzylidene)amino]-6-methyl-3-(pyridin-3-ylmethylsulfanyl)-4*H*-[1,2,4]triazin-5-one
and physiologically acceptable salts and solvates thereof.

7. Process for the preparation of compounds of the formula I according to Claim 1 and salts and solvates thereof, **characterised in that** a compound of the formula II 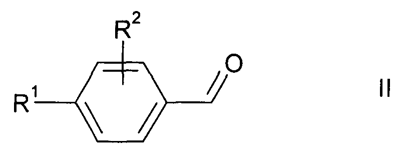 in which
R¹ and R² have the meanings indicated,
is reacted with a compound of the formula III 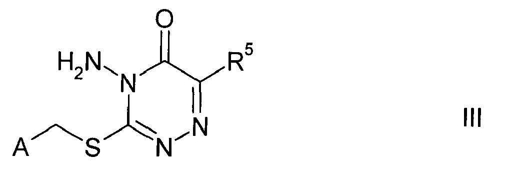 in which
A and R⁵ have the meanings indicated in Claim 1,
and/or **in that** a basic compound of the formula I is converted into one of its salts by treatment with an acid.

8. Compounds of the formula III 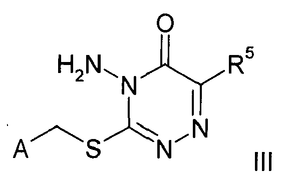 in which A and R⁵ are as defined in Claim 1, with the proviso that compounds of the formula III in which A is phenyl and R³ and R⁴ are both H, or one of the two is Cl, or A is 2-pyridyl, are excluded.

9. Compounds of the formula I according to one or more of Claims 1 to 6 and physiologically acceptable salts and solvates thereof as medicaments.

10. Use of the compounds of the formula I according to one or more of Claims 1 to 6 and/or physiologically acceptable salts and/or solvates thereof for the preparation of a medicament.

11. Compounds of the formula I according to one or more of Claims 1 to 6 and physiologically acceptable salts and solvates thereof as phosphodiesterase IV inhibitors.

12. Pharmaceutical preparation, **characterised by** a content of at least one compound of the formula I according to one or more of Claims 1 to 6 and/or one of its physiologically acceptable salts and/or one of its solvates.

13. Process for the preparation of pharmaceutical preparations, **characterised in that** a compound of the formula I according to one or more of Claims 1 to 6 and/or one of its physiologically acceptable salts and/or one of its solvates is brought into a suitable dosage form together with at least one solid, liquid or semi-liquid excipient or adjuvant.

14. Use of the compounds of the formula I according to one or more of Claims 1 to 6 and/or physiologically acceptable salts and/or solvates thereof for the preparation of a medicament for the treatment and prophylaxis of heart diseases (myocardial diseases).

15. Use of compounds of the formula I according to one or more of Claims 1 to 6 and/or salts and/or solvates thereof for the preparation of a medicament for the treatment of myocardial diseases having inflammatory and immunological characteristics.

16. Use of the compounds of the formula I according to one or more of Claims 1 to 6 and/or salts and/or solvates thereof for the preparation of a medicament for the treatment of coronary heart disease, reversible or irreversible myocardial ischaemia/reperfusion damage, acute or chronic heart failure and restenosis, including in-stent restenosis and stent-in-stent restenosis.

17. Use of the compounds of the formula I according to one or more of Claims 1 to 6 and/or physiologically acceptable salts and/or solvates thereof for the preparation of a medicament for the prophylaxis and treatment of ventricular remodelling after infarction or decompensated cardiac insufficiency (congestive heart failure, CHF) of varying severity.

18. Use of the compounds of the formula I according to one or more of Claims 1 to 6 and/or physiologically acceptable salts and/or solvates thereof for the preparation of a medicament for the treatment and prophylaxis of diseases which are caused by an excessively low cAMP level and/or can be influenced by an increase in the cAMP level.

19. Use of the compounds of the formula I according to one or more of Claims 1 to 6 and/or physiologically acceptable salts and/or solvates thereof for the preparation of a medicament for the treatment and prophylaxis of diseases which can be influenced by a reduction in the production of tumour necrosis factor (TNF).

20. Use of the compounds of the formula I according to one or more of Claims 1 to 6 and/or physiologically acceptable salts and/or solvates thereof for the preparation of a medicament for the treatment and prophylaxis of diseases which are caused by overproduction of macrophages and T-cells and/or can be influenced by a reduction in macrophage and T-cell production.

21. Use of the compounds of the formula I according to one or more of Claims 1 to 6 and/or physiologically acceptable salts and/or solvates thereof for the preparation of a medicament for the treatment and prophylaxis of diseases which are caused by excessive proliferation of the T-cells and/or can be influenced by inhibition of the proliferation of the T-cells.

22. Use of the compounds of the formula I according to one or more of Claims 1 to 6 and/or physiologically tolerated salts and/or solvates thereof for the preparation of a medicament for the treatment or prophylaxis of diseases which are caused by disurbances in the regulation of the activation and degranulation of human eosinophiles.

23. Compounds of the formula I according to one or more of Claims 1 to 6 and/or physiologically acceptable salts and/or solvates thereof for combating allergic diseases, asthma, chronic bronchitis, atopic dermatitis, psoriasis and other skin diseases, inflammatory diseases, autoimmune diseases, such as, for example, rheumatoid arthritis, multiple sclerosis, Crohn's disease, diabetes mellitus or ulcerative colitis, osteoporosis, transplant rejection reactions, cachexia, tumour growth or tumour metastases, sepsis, memory disorders, atherosclerosis and AIDS.

24. Use of compounds of the formula I according to one or more of Claims 1 to 6 and/or physiologically acceptable salts and/or solvates thereof for the preparation of a medicament for combating allergic diseases, asthma, chronic bronchitis, atopic dermatitis, psoriasis and other skin diseases, inflammatory diseases, autoimmune diseases, such as, for example, rheumatoid arthritis, multiple sclerosis, Crohn's disease, diabetes mellitus or ulcerative colitis, osteoporosis, transplant rejection reactions, cachexia, tumour growth or tumour metastases, sepsis, memory disorders, atherosclerosis and AIDS.

25. Use of compounds of the formula III 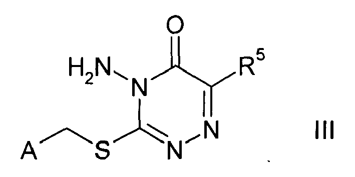 in which A and R⁵ are as defined in Claim 1, for the preparation of compounds of the formula I according to Claim 1.

## Revendications

1. Composés de la formule I 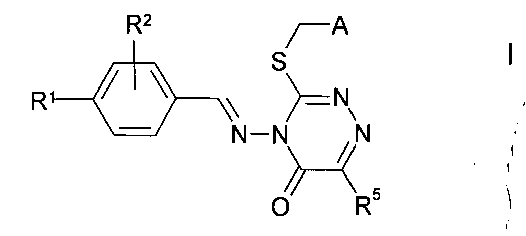 dans laquelle
R¹, R² chacun indépendamment l'un de l'autre, représentent H, OH, OR⁶ SR⁶, SOR⁶, SO₂R⁶, Hal ou représentent ensemble également -O-CH₂-O-,
A représente phényle substitué par R³ et R⁴, 2-, 3- ou 4-pyridyle, 4- ou 5-pyrimidyle, 3- ou 4-pyridazyle, 2- ou 3-pyrazinyle,
R³, R⁴ chacun indépendamment l'un de l'autre, représentent H, OH, OR⁶ SR⁶, SOR⁶, SO₂R⁶, R⁶, Hal ou représentent ensemble également -O-CH₂-O-,
R⁵ représente H ou alkyle comportant de 1 à 10 atomes de C,
R⁶ représente alkyle comportant de 1 à 10 atomes de C, qui peut être substitué par 1 à 5 atomes de F et/ou de Cl, cycloalkyle comportant de 3 à 7 atomes de C, alkylène-cycloalkyle comportant de 5 à 10 atomes de C ou alkényle comportant de 2 à 8 atomes de C,
Hal représente F, Cl, Br ou I,
et leur sels et solvates physiologiquement acceptables.

2. Composés de la formule I selon la revendication 1, dans lesquels R⁵ présente la signification méthyle.

3. Composés de la formule I selon une ou plusieurs des revendications 1 et 2, dans lesquels R¹ et R², chacun indépendamment l'un de l'autre, représentent OR⁶.

4. Composés de la formule I selon une ou plusieurs des revendications 1 à 3, dans lesquels R⁶ représente alkyle comportant de 1 à 10 atomes de C ou cycloalkyle comportant de 3 à 7 atomes de C.

5. Composés de la formule I selon une ou plusieurs des revendications 1 à 4, dans lesquels A représente phényle, 2-, 3- ou 4-pyridyle, 4- ou 5-pyrimidyle et R³ et R⁴, chacun indépendamment l'un de l'autre, représentent R⁶, H, Cl, F, CF₃ ou OR⁶.

6. Composés des formules (a) à (h) selon la revendication 1 :
(a) 4-[(3-éthoxy-4-méthoxybenzylidène)amino]-3-(benzylsulfanyl)-6-méthyl-4*H*-[1,2,4]triazine-5-one
(b) 4-[(3-éthoxy-4-méthoxybenzylidène)amino]-3-(2-fluorobenzylsulfanyl)-6-méthyl-4*H*-[1,2,4]triazine-5-one
(c) 4-[(3-éthoxy-4-méthoxybenzylidène)amino]-3-(2-chloro-6-fluorobenzylsulfanyl)-6-méthyl-4*H*-[1,2,4]triazine-5-one
(d) 4-[(3-éthoxy-4-méthoxybenzylidène)amino]-6-méthyl-3-(pyridine-3-ylméthylsulfanyl)-4*H*-[1,2,4]triazine-5-one
(e) 4-[(3-cyclopentyloxy-4-méthoxybenzylidène)amino]-3-(benzylsulfanyl)-6-méthyl-4*H*-[1,2,4]triazine-5-one
(f) 4-[(3-cyclopentyloxy-4-méthoxybenzylidène)amino]-3-(2-fluorobenzylsulfanyl)-6-méthyl-4*H*-[1,2,4]triazine-5-one
(g) 4-[(3-cyclopentyloxy-4-méthoxybenzylidène)amino]-3-(2-chloro-6-fluorobenzylsulfanyl)-6-méthyl-4*H*-[1,2,4]triazine-5-one
(h) 4-[(3-cyclopentyloxy-4-méthoxybenzylidène)amino]-6-méthyl-3-(pyridine-3-ylméthylsulfanyl)-4*H*-[1,2,4]triazine-5-one
et leur sels et solvates physiologiquement acceptables.

7. Procédé pour la préparation de composés de la formule I selon la revendication 1 et de leur sels et solvates, **caractérisé en ce qu'**un composé de la formule II 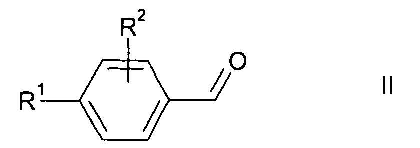 dans laquelle
R¹ et R² ont les significations indiquées,
est amené à réagir avec un composé de la formule III 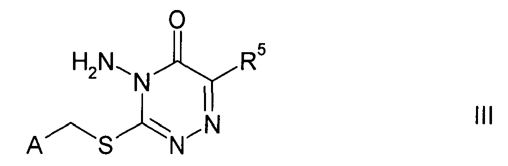 dans laquelle
A et R⁵ ont les significations indiquées dans la revendication 1,
et/ou **en ce qu'**un composé basique de la formule I est converti selon l'un de ses sels par traitement avec un acide.

8. Composés de la formule III 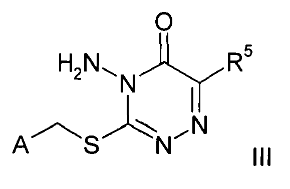 dans laquelle A et R⁵ sont comme défini dans la revendication 1, étant entendu que des composés de la formule III dans laquelle A est phényle et R³ et R⁴ sont tous deux H ou l'un des deux est Cl, ou A est 2-pyridyle, sont exclus.

9. Composés de la formule I selon une ou plusieurs des revendications 1 à 6 et leur sels et solvates physiologiquement acceptables en tant que médicaments.

10. Utilisation des composés de la formule I selon une ou plusieurs des revendications 1 à 6 et/ou de leur sels et/ou solvates physiologiquement acceptables pour la préparation d'un médicament.

11. Composés de la formule I selon une ou plusieurs des revendications 1 à 6 et leur sels et solvates physiologiquement acceptables en tant qu'inhibiteurs de phosphodiestérase IV.

12. Préparation pharmaceutique, **caractérisée par** une teneur en au moins un composé de la formule I selon une ou plusieurs des revendications 1 à 6 et/ou en un de leurs sels et/ou en un de leurs solvates physiologiquement acceptables.

13. Procédé pour la préparation de préparations pharmaceutiques, **caractérisé en ce qu'**un composé de la formule I selon une ou plusieurs des revendications 1 à 6 et/ou un de ses sels et/ou un de ses solvates physiologiquement acceptables est amené selon une forme de dosage appropriée en association avec au moins un excipient ou adjuvant solide, liquide ou semi-liquide.

14. Utilisation des composés de la formule I selon une ou plusieurs des revendications 1 à 6 et/ou de leur sels et/ou solvates physiologiquement acceptables pour la préparation d'un médicament pour le traitement et la prophylaxie de maladies du coeur (maladies du myocarde).

15. Utilisation de composés de la formule I selon une ou plusieurs des revendications 1 à 6 et/ou de leur sels et/ou solvates pour la préparation d'un médicament pour le traitement de maladies du myocarde présentant des caractéristiques inflammatoires et immunologiques.

16. Utilisation des composés de la formule I selon une ou plusieurs des revendications 1 à 6 et/ou de leur sels et/ou solvates pour la préparation d'un médicament pour le traitement d'une maladie des coronaires du coeur, d'un dommage réversible ou irréversible par ischémie/reperfusion myocardiale, d'une défaillance et d'une resténose cardiaques aiguës ou chroniques, incluant une resténose intra-stent et une resténose stent-intra-stent.

17. Utilisation des composés de la formule I selon une ou plusieurs des revendications 1 à 6 et/ou de leur sels et/ou solvates physiologiquement acceptables pour la préparation d'un médicament pour la prophylaxie et le traitement du remodelage ventriculaire après infarctus ou insuffisance cardiaque décompensée (défaillance cardiaque congestive, DCC) de sévérité variable.

18. Utilisation des composés de la formule I selon une ou plusieurs des revendications 1 à 6 et/ou de leur sels et/ou solvates physiologiquement acceptables pour la préparation d'un médicament pour le traitement et la prophylaxie de maladies qui sont générées par un niveau cAMP excessivement faible et/ou qui peuvent être influencées par une augmentation du niveau cAMP.

19. Utilisation des composés de la formule I selon une ou plusieurs des revendications 1 à 6 et/ou de leur sels et/ou solvates physiologiquement acceptables pour la préparation d'un médicament pour le traitement et la prophylaxie de maladies qui peuvent être influencées par une réduction de la production d'un facteur de nécrose de tumeur (FNT).

20. Utilisation des composés de la formule I selon une ou plusieurs des revendications 1 à 6 et/ou de leur sels et/ou solvates physiologiquement acceptables pour la préparation d'un médicament pour le traitement et la prophylaxie de maladies qui sont générées par une surproduction de macrophages et de cellules T et/ou qui peuvent être influencées par une réduction de la production de macrophages et de cellules T.

21. Utilisation des composés de la formule I selon une ou plusieurs des revendications 1 à 6 et/ou de leurs sels et/ou solvates physiologiquement acceptables pour la préparation d'un médicament pour le traitement et la prophylaxie de maladies qui sont générées par une prolifération excessive des cellules T et/ou qui peuvent être influencées par une inhibition de la prolifération des cellules T.

22. Utilisation des composés de la formule I selon une ou plusieurs des revendications 1 à 6 et/ou de leurs sels et/ou solvates physiologiquement tolérés pour la préparation d'un médicament pour le traitement ou la prophylaxie de maladies qui sont générées par des troubles de la régulation de l'activation et de la dégranulation des éosinophiles humains.

23. Composés de la formule I selon une ou plusieurs des revendications 1 à 6 et/ou leurs sels et/ou solvates physiologiquement acceptables pour combattre les maladies allergiques, l'asthme, la bronchite chronique, la dermatite atopique, le psoriasis et d'autres maladies de la peau, les maladies inflammatoires, les maladies auto-immunes telles que, par exemple, l'arthrite rhumatoïde, la sclérose en plaques, la maladie de Crohn, le diabète sucré ou la recto-colite hémorragique, l'ostéoporose, les réactions de rejet de greffon, le marasme, la croissance de tumeur ou les métastases de tumeur, la sepsie, les troubles de la mémoire, l'athérosclérose et le SIDA.

24. Utilisation de composés de la formule I selon une ou plusieurs des revendications 1 à 6 et/ou de leurs sels et/ou solvates physiologiquement acceptables pour la préparation d'un médicament pour combattre les maladies allergiques, l'asthme, la bronchite chronique, la dermatite atopique, le psoriasis et d'autres maladies de la peau, les maladies inflammatoires, les maladies auto-immunes telles que, par exemple, l'arthrite rhumatoïde, la sclérose en plaques, la maladie de Crohn, le diabète sucré ou la recto-colite hémorragique, l'ostéoporose, les réactions de rejet de greffon, le marasme, la croissance de tumeurs ou les métastases de tumeurs, la sepsie, les troubles de la mémoire, l'athérosclérose et le SIDA.

25. Utilisation de composés de la formule III 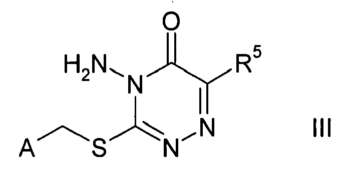 dans laquelle A et R⁵ sont comme défini dans la revendication 1, pour la préparation de composés de la formule 1 selon la revendication 1.
